# EUROPEAN PATENT APPLICATION

(11) **EP 4 435 010 A1**
(43) Date of publication of application: **25.09.2024**
(21) Application number: 22894869.1
(22) Date of filing: 17.11.2022
(51) Int. Cl.: C07K 16/46, C12N 15/13, C12N 15/62, C12N 15/63, A61K 39/395, A61P 35/00

(54) **BISPECIFIC ANTIBODY AGAINST TIGIT AND PD-L1, AND PHARMACEUTICAL COMPOSITION THEREOF AND USE THEREOF**

(30) Priority: 17.11.2021 CN 202111359665
(71) Applicant: Biotheus Inc., Tangjiawan Town, Xiangzhou District Zhuhai, Guangdong 519080 (CN)
(72) Inventor: DAI, Shuang, Zhuhai, Guangdong 519080 (CN); ZHAI, Tianhang, Zhuhai, Guangdong 519080 (CN); HUANG, Weifeng, Zhuhai, Guangdong 519080 (CN); PENG, Shaogang, Zhuhai, Guangdong 519080 (CN); SUN, Tsoyue Joanne, Zhuhai, Guangdong 519080 (CN)
(74) Representative: Casci, Tamara
(86) International application number: PCT/CN2022/132422
(87) International publication number: WO 2023/088337

(57) **Abstract**

The present invention belongs to the field of biomedicine, and relates to a bispecific antibody against TIGIT and PD-L1, and a pharmaceutical composition thereof and the use thereof. The bispecific antibody of the present invention comprises: a TIGIT-targeting first protein functional region and a PD-L1-targeting second protein functional region, wherein the first protein functional region is an anti-TIGIT antibody or an antigen-binding fragment thereof, the heavy chain variable region of the anti-TIGIT antibody contains HCDR1 having an amino acid sequence as shown in SEQ ID NO: 10, HCDR2 having an amino acid sequence as shown in SEQ ID NO: 11 and HCDR3 having an amino acid sequence as shown in SEQ ID NO: 12, and the light chain variable region of the anti-TIGIT antibody contains LCDR1 having an amino acid sequence as shown in SEQ ID NO: 13, LCDR2 having an amino acid sequence as shown in SEQ ID NO: 14 and LCDR3 having an amino acid sequence as shown in SEQ ID NO: 15. The bispecific antibody of the present invention has high affinity to dual targets, i.e. TIGIT and PD-L1, and has good application prospects.

## Description

### Technical Field

The present invention belongs to the field of medicine and relates to an anti-TIGIT/anti-PD-L1 bispecific antibody, its pharmaceutical composition and uses.

### Background Art

T cell immunoreceptor with Ig and ITIM domains (TIGIT, also known as WUCAM, Vstm3, VSIG9) is a new type of immunosuppressive receptor expressed by activated CD8⁺ T and CD4⁺ T cells, natural killer (NK) cells, regulatory T cells (Tregs) and follicular helper T cells.

TIGIT participates in a complex regulatory network in tumor immunity, involving multiple immunosuppressive receptors (e.g., CD96/TACTILE, CD112R/PVRIG), a competitive costimulatory receptor (DNAM-1/CD226), and multiple ligands (e.g., CD155 (PVR/NECL-5), CD112 (Nectin-2/PVRL2)). DNAM-1, TIGIT and CD96 are expressed on T cells and NK cells and share CD155 in the form of ligand.

The role of TIGIT in tumor immunosuppression is similar to that of PD-1/PD-L1. Current research has proposed several mechanisms of TIGIT-mediated inhibition of effector T cells and NK cells: (1) the binding of TIGIT on the surface of T/NK cells to CD155 phosphorylates the immunoreceptor tyrosine inhibitory motif (ITIM) in TIGIT cells, and directly transduces inhibitory signals; (2) the binding of TIGIT to CD155 on DCs promotes the generation of immune-tolerant DCs, reduces the production of interleukin (IL)-12 and the increases of IL-10, and indirectly inhibits T cell responses; (3) TIGIT, which has an inhibitory effect, competitively binds to CD155 with a higher affinity than that of the costimulatory receptor CD226, thereby limiting the CD226-mediated activation; in addition, TIGIT also directly binds to CD226 in a cis manner on cells, which destroys its ability to bind to the homodimer of CD155; (4) Tregs expressing TIGIT are highly inhibitory, and TIGIT+Tregs produce IL-10 and fibrinogen-like protein 2 (Fgl2) upon activation, which mediate the T cell inhibition; (5) Fap2 protein from *Fusobacterium nucleatum* (an anaerobic Gram-symbiotic bacterium implicated in colorectal cancer) can directly bind to TIGIT, but does not bind to CD226, thereby inhibiting tumor immunity mediated by NK cells and T cells, and regulating innate immune responses.

Programmed death 1 ligand 1 (PD-L1), also known as CD274, is a member of the B7 family, and is a ligand of PD-1. PD-L1 is a type I transmembrane protein with a total of 290 amino acids, comprising one IgV-like region, one IgC-like region, one transmembrane hydrophobic region, and one intracellular region composed of 30 amino acids. Unlike other B7 family molecules, PD-L1 has the effect of negatively regulating immune responses. Studies have found that PD-L1 is mainly expressed in activated T cells, B cells, macrophages and dendritic cells. In addition to lymphocytes, PD-L1 is also expressed in endothelial cells of various other tissues such as thymus, heart, placenta, etc., and various types of non-lymphoid systems, such as melanoma, liver cancer, gastric cancer, renal cell carcinoma, ovarian cancer, colon cancer, breast cancer, esophageal cancer, head and neck cancer, etc. (Akintunde Akinleye & Zoaib Rasool, Journal of Hematology & Oncology volume 12, Article number: 92 (2019)). PD-L1 has a broad spectrum in regulating autoreactive T and B cells and immune tolerance, and plays a role in T and B cell responses in peripheral tissue. High expression of PD-L1 on tumor cells is associated with poor prognosis in cancer patients.

Although bispecific antibodies are a direction for antibody drug development, they face many challenges, such as preclinical evaluation models, low expression levels, poor stability, complex processes, and large differences in quality control. Therefore, the research and development of bispecific antibodies have always been fraught with difficulties.

Therefore, there is a need to develop a bispecific antibody targeting TIGIT and PD-L1 that has good specificity, good efficacy and is easy to prepare.

### Contents of the present invention

After in-depth research and creative work, the inventors obtained an anti-TIGIT antibody and constructed an anti-TIGIT/anti-PD-L1 bispecific antibody based on the antibody. The inventors surprisingly found that the anti-TIGIT antibody (also referred to as the antibody or the antibody of the present invention) and the anti-TIGIT/anti-PD-L1 bispecific antibody (also referred to as the bispecific antibody or the bispecific antibody of the present invention) of the present invention have high binding affinity to PD-L1 and TIGIT (the bispecific antibody is even better than the positive control antibody in one or more aspects), can block the binding of PD-L1 to its ligand PD-1 and the binding of TIGIT to its ligand CD155/CD112, respectively, thereby reducing or eliminating the inhibitory signal transmitted to cells; and the administration of the antibody of the present invention can significantly inhibit tumor growth in animal models. The following invention is thereby provided:
One aspect of the present invention designs a bispecific antibody, which comprises:
a first protein functional region targeting TIGIT, and
a second protein functional region targeting a target (such as PD-L1) different from TIGIT;
wherein:
   the first protein functional region is an anti-TIGIT immunoglobulin or an antigen-binding fragment thereof;
   the heavy chain variable region of the anti-TIGIT immunoglobulin comprises HCDR1 with the amino acid sequence as set forth in SEQ ID NO: 10, HCDR2 with the amino acid sequence as set forth in SEQ ID NO: 11, and HCDR3 with the amino acid sequence as set forth in SEQ ID NO: 12; and
   the light chain variable region of the anti-TIGIT immunoglobulin comprises LCDR1 with the amino acid sequence as set forth in SEQ ID NO: 13, LCDR2 with the amino acid sequence as set forth in SEQ ID NO: 14, and LCDR3 with the amino acid sequence as set forth in SEQ ID NO: 15.

The variable regions of the light chain and the heavy chain determine the binding to antigen; the variable region of each chain comprises three hypervariable regions, called complementarity determining regions (CDRs), of which the CDRs of the heavy chain (H) include HCDR1, HCDR2, and HCDR3, and the CDRs of the light chain (L) include LCDR1, LCDR2, and LCDR3. The CDRs contained in the antibody or an antigen-binding fragment thereof of the present invention can be determined according to various numbering systems known in the art. In certain embodiments, the CDRs contained in the antibody or an antigen-binding fragment thereof of the present invention are preferably determined by the Kabat, Chothia, AbM HVR or IMGT numbering system. Unless otherwise stated, the CDRs contained in the antibody or an antigen-binding fragment thereof of the present invention are preferably determined by the Kabat numbering system (Kabat et al., Sequences of Proteins of Immunological Interest, 5th Ed. Public Health Service, National Institute of Health, Bethesda, Md.(1991)).

In some embodiments of the present invention, in the bispecific antibody, the first protein functional region and the second protein functional region are directly linked or linked via a linker (also called a linker peptide);
preferably, the linker is (GGGGS)ₘ, and m is a positive integer, such as 1, 2, 3, 4, 5 or 6;
preferably, the linker is (GGGGS)ₙG, n is a positive integer, such as 1, 2, 3, 4, 5 or 6;
preferably, the amino acid sequence of the linker is set forth in SEQ ID NO: 2;
wherein, GGGGS in brackets is represented by SEQ ID NO: 22.

In some embodiments of the present invention, in the bispecific antibody, the numbers of the first protein functional region and the second protein functional region are independently 1, 2, or more than 2.

In some embodiments of the present invention, in the bispecific antibody, the number of the first protein functional region is 1, and the number of the second protein functional region is 2.

In some embodiments of the present invention, in the bispecific antibody, the number of the first protein functional region is 2, and the number of the second protein functional region is 1.

In some embodiments of the present invention, in the bispecific antibody,
the first protein functional region is an anti-TIGIT immunoglobulin or an antigen-binding fragment thereof, and the second protein functional region is a single-domain antibody or a single-chain antibody targeting a target different from TIGIT;
preferably, the single-domain antibody is an anti-PD-L1 single-domain antibody;
preferably, the single-chain antibody is an anti-PD-L1 single-chain antibody.

In some embodiments of the present invention, in the bispecific antibody,
the first protein functional region is an anti-TIGIT immunoglobulin or an antigen-binding fragment thereof, and the second protein functional region is an anti-PD-L1 single-domain antibody or an anti-PD-L1 single-chain antibody.

In some embodiments of the present invention, in the bispecific antibody,
the first protein functional region is an anti-TIGIT single-chain antibody, and the second protein functional region is an immunoglobulin or an antigen-binding fragment thereof of which the target is different from TIGIT;
preferably, the immunoglobulin targeting a target different from TIGIT is an anti-PD-L1 immunoglobulin.

In some embodiments of the present invention, in the bispecific antibody,
the first protein functional region is an anti-TIGIT single-chain antibody, and the second protein functional region is an anti-PD-L1 immunoglobulin or an antigen-binding fragment thereof.

In some embodiments of the present invention, in the bispecific antibody,
the anti-PD-L1 single-domain antibody comprises one heavy chain variable region, and the heavy chain variable region comprises HCDR1 with the amino acid sequence as set forth in SEQ ID NO: 16, HCDR2 with the amino acid sequence as set forth in SEQ ID NO: 17, and HCDR3 with the amino acid sequence as set forth in SEQ ID NO: 18;
preferably, the amino acid sequence of the anti-PD-L1 single-domain antibody is set forth in SEQ ID NO: 3.

In some embodiments of the present invention, in the bispecific antibody,
in the anti-TIGIT immunoglobulin, the amino acid sequence of the heavy chain variable region is set forth in SEQ ID NO: 19, and the amino acid sequence of the light chain variable region is set forth in SEQ ID NO: 20;
preferably,
in the anti-TIGIT immunoglobulin, the amino acid sequence of the heavy chain is set forth in SEQ ID NO: 1, and the amino acid sequence of the light chain is set forth in SEQ ID NO: 5.

In some embodiments of the present invention, in the bispecific antibody, the antigen-binding fragment is selected from the group consisting of Fab, Fab', F(ab')₂, Fd, Fv, dAb and complementarity determining region fragments.

In some embodiments of the present invention, in the bispecific antibody,
the constant region of the anti-TIGIT immunoglobulin or of the immunoglobulin targeting a target different from TIGIT is derived from a human antibody;
preferably, the constant region is selected from the constant regions of human IgGl, IgG2, IgG3 or IgG4.

In some embodiments of the present invention, in the bispecific antibody,
in the anti-TIGIT immunoglobulin or the immunoglobulin targeting a target different from TIGIT, the heavy chain constant region is a human Ig gamma-1 chain C region (e.g., NCBI ACCESSION: P01857) or a human Ig gamma-4 chain C region (e.g., NCBI ACCESSION: P01861.1), and the light chain constant region is a human Ig kappa chain C region (e.g., NCBI ACCESSION: P01834); preferably, the heavy chain constant region of the anti-TIGIT immunoglobulin also comprises a L234A mutation and a L235A mutation according to the EU numbering system.

In some embodiments of the present invention, in the bispecific antibody, the single-domain antibody or single-chain antibody is linked to C-terminal or N-terminal of the anti-TIGIT immunoglobulin, for example, the number of the single-domain antibody or single-chain antibody is 2, and one end of each single-domain antibody or single-chain antibody is linked to C-terminal or N-terminal of the two heavy chains of the anti-TIGIT immunoglobulin, respectively.

In some embodiments of the present invention, in the bispecific antibody, the anti-PD-L1 single-domain antibody or anti-PD-L1 single-chain antibody is linked to C-terminal or N-terminal of the anti-TIGIT immunoglobulin, for example, the number of the anti-PD-L1 single-domain antibody or anti-PD-L1 single-chain antibody is 2, and one end of each anti-PD-L1 single-domain antibody or anti-PD-L1 single-chain antibody is linked to C-terminal or N-terminal of the two heavy chains of the anti-TIGIT immunoglobulin, respectively.

In some embodiments of the present invention, in the bispecific antibody, the single-domain antibody is an anti-PD-L1 single-domain antibody, and the peptide chain obtained by linking the single-domain antibody to the anti-TIGIT immunoglobulin has the amino acid sequence as set forth in SEQ ID NO: 4.

In some embodiments of the present invention, the bispecific antibody is a tetramer formed by two identical first peptide chains and two identical second peptide chains, wherein,
the amino acid sequence of the first peptide chain is set forth in SEQ ID NO: 4; and the amino acid sequence of the second peptide chain is set forth in SEQ ID NO: 5.

In some embodiments of the present invention, the bispecific antibody comprises:
a first protein functional region targeting TIGIT, and
a second protein functional region targeting PD-L1;
the number of the first protein functional region is 1, and the number of the second protein functional region is 2;
wherein, the first protein functional region is an anti-TIGIT immunoglobulin, and the second protein functional region is an anti-PD-L1 single-domain antibody;
in the anti-TIGIT immunoglobulin, the amino acid sequence of the heavy chain is set forth in SEQ ID NO: 1, and the amino acid sequence of the light chain is set forth in SEQ ID NO: 5;
the amino acid sequence of the anti-PD-L1 single-domain antibody is set forth in SEQ ID NO: 3;
the two anti-PD-L1 single domain antibodies are linked to C-terminal of each heavy chain of the anti-TIGIT immunoglobulin through the same or different linker, respectively;
preferably, the linker is (GGGGS)ₘ, and m is a positive integer, such as 1, 2, 3, 4, 5 or 6;
preferably, the linker is (GGGGS)ₙG, and n is a positive integer, such as 1, 2, 3, 4, 5 or 6;
preferably, the amino acid sequence of the linker is set forth in SEQ ID NO: 2;
wherein, GGGGS in the brackets is represented by SEQ ID NO: 22.

In one or more embodiments of the present invention, the bispecific antibody is in the form of IgG-VHH.

In one or more embodiments of the present invention, the bispecific antibody is in the form of IgG-scFv, that is, Morrison pattern.

In one or more embodiments of the present invention, in the bispecific antibody, the single-domain antibody or single-chain antibody is linked to C-terminal of the heavy chain of immunoglobulin. Since immunoglobulin consists of two heavy chains, one immunoglobulin molecule is linked to two single-domain antibody molecules or two single-chain antibody molecules. Preferably, the two single-domain antibody molecules are identical. Preferably, the two single-chain antibody molecules are identical. Preferably, the single-domain antibody or single-chain antibody is linked to C-terminal of the heavy chain of the immunoglobulin through an amide bond formed by the aforementioned linker.

In one or more embodiments of the present invention, for the bispecific antibody, its monovalent affinity for binding to human PD-L1, cynomolgus monkey PD-L1, human TIGIT and/or cynomolgus monkey TIGIT antigens, respectively, is equivalent to better than the monovalent affinity of its single-end antibody molecule for binding to human PD-L1, cynomolgus monky PD-L1, human TIGIT and/or cynomolgus monkey TIGIT anti-antigens.

Another aspect of the present invention relates to an isolated nucleic acid molecule, which encodes the bispecific antibody according to any one of the items of the present invention.

A further aspect of the present invention relates to a vector, which comprises the isolated nucleic acid molecule of the present invention.

A further aspect of the present invention relates to a host cell, which comprises the isolated nucleic acid molecule of the present invention, or the vector of the present invention.

A further aspect of the present invention relates to a method for preparing the bispecific antibody according to any one of the items of the present invention, which comprises steps of culturing the host cell of the present invention under suitable conditions, and recovering the bispecific antibody from a cell culture.

Another aspect of the present invention relates to a conjugate, which comprises the bispecific antibody and a conjugation moiety, wherein the bispecific antibody is the bispecific antibody according to any one of the items of the present invention, and the conjugation moiety is a detectable label; preferably, the conjugation moiety is a radioactive isotope, a fluorescent substance, a luminescent substance, a colored substance or an enzyme.

Another aspect of the present invention relates to a kit, which comprises the bispecific antibody according to any one of the items of the present invention, or the conjugate of the present invention;
preferably, the kit further comprises a second antibody that is capable of specifically binding to the bispecific antibody; optionally, the second antibody further comprises a detectable label, such as a radioactive isotope, a fluorescent substance, a luminescent substance, a colored substance or an enzyme.

Another aspect of the present invention relates to a pharmaceutical composition, which comprises the bispecific antibody according to any one of the items of the present invention or the conjugate of the present invention; optionally, the pharmaceutical composition further comprises a pharmaceutically acceptable excipient.

Another aspect of the present invention relates to a use of the bispecific antibody of any one of the items of the present invention or the conjugate of the present invention in the manufacture of a medicament for preventing and/or treating a malignant tumor; preferably, the malignant tumor is are selected from the group consisting of melanoma, liver cancer, stomach cancer, renal cell cancer, ovarian cancer, colon cancer, breast cancer, esophageal cancer, and head and neck cancer.

Yet another aspect of the present invention relates to a method for treating and/or preventing a malignant tumor, comprising a step of administering to a subject in need thereof an effective amount of the bispecific antibody of any one of the items of the present invention or the conjugate of the present invention; preferably, the malignant tumor is selected from the group consisting of melanoma, liver cancer, gastric cancer, renal cell carcinoma, ovarian cancer, colon cancer, breast cancer, esophageal cancer, and head and neck cancer.

In some embodiments of the present invention, in the method for treating and/or preventing the malignant tumor, the step of administering to the subject in need thereof an effective amount of the bispecific antibody of any one of the items of the present invention is performed before or after a surgical treatment, and/or before or after a radiation therapy.

In some embodiments of the present invention, in the method of treating and/or preventing the malignant tumor,
the single dosage of the bispecific antibody of the present invention is 0.1 to 100 mg, preferably 4.8 to 24 mg or 1 to 10 mg, per kilogram of body weight; alternatively, the single dosage of the bispecific antibody of the present invention is 10 to 1000 mg, preferably 50 to 500mg, 100 to 400mg, 150 to 300mg, 150 to 250mg, or 200mg, per kilogram of body weight;
preferably, the administering is performed every 3 days, 4 days, 5 days, 6 days, 10 days, 1 week, 2 weeks or 3 weeks;
preferably, the administering is performed by the manner of intravenous drip or intravenous injection.

The bispecific antibody according to any one of the items of the present invention or the conjugate of the present invention, which is used for treating and/or preventing a malignant tumor; preferably, the malignant tumor is selected from the group consisting of melanoma, liver cancer, gastric cancer, renal cell carcinoma, ovarian cancer, colon cancer, breast cancer, esophageal cancer, and head and neck cancer.

In the present invention, unless otherwise stated, scientific and technical terms used herein have the meanings commonly understood by those skilled in the art. Moreover, the procedures of cell culture, molecular genetics, nucleic acid chemistry, and immunology laboratory used herein are routine procedures widely used in the corresponding fields. Meanwhile, in order to better understand the present invention, definitions and explanations of relevant terms are provided below.

The term "EC₅₀" refers to a concentration for 50% of maximal effect, which refers to a concentration that causes 50% of maximum effect.

The term "antibody" refers to an immunoglobulin molecule usually composed of two pairs of polypeptide chains, each pair having a "light" (L) chain and a "heavy" (H) chain. Antibody light chains can be classified into κ and λ light chains. Heavy chains can be classified as µ, δ, γ, α, or ε, and define the antibody's isotype as IgM, IgD, IgG, IgA, and IgE, respectively. Within the light and heavy chains, the variable and constant regions are connected by a "J" region of approximately 12 or more amino acids, and the heavy chain also contains a "D" region of approximately 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. The constant region of antibody may mediate the binding of immunoglobulin to host tissues or factors, including various cells (e.g., effector cells) of immune system and first component (Clq) of classical complement system. The VH and VL regions can also be subdivided into highly variable regions called complementarity determining regions (CDRs), interspersed therewith more conservative regions called framework regions (FRs). Each VH and VL consists of 3 CDRs and 4 FRs arranged from the amino terminus to the carboxyl terminus in the following order: FR1, CDR1, FR2, CDR2, FR3, CDR3 and FR4. The variable regions (VH and VL) of each heavy chain/light chain pair respectively form an antibody binding site. The assignment of amino acids to regions or domains follows the definition of Bethesda M.d., Kabat Sequences of Proteins of Immunological Interest (National Institutes of Health, (1987 and 1991)), or Chothia & Lesk J. Mol. Biol. 1987; 196: 901-917; Chothia et al, Nature, 1989; 342: 878-883; or the IMGT numbering system, see, the definition of Ehrenmann F, Kaas Q, Lefranc M P. IMGT/3Dstructure-DB and IMGT/DomainGapAlign: a database and a tool for immunoglobulins or antibodies, T cell receptors, MHC, IgSF and MhcSF [J]. Nucleic acids research, 2009; 38(suppl_1): of D301-D307.

The term "antibody" is not limited to any particular method of producing antibody, which includes, for example, recombinant antibodies, monoclonal antibodies, and polyclonal antibodies. Antibodies may be of different isotypes, for example, IgG (e.g., IgGl, IgG2, IgG3 or IgG4 subtype), IgA1, IgA2, IgD, IgE or IgM antibody.

The terms "mcAb" and "monoclonal antibody" refer to an antibody or a fragment of antibody from a group of highly homologous antibody molecules, that is, a group of identical antibody molecules except for natural mutations that may occur spontaneously. Monoclonal antibody is highly specific for a single epitope on an antigen. Polyclonal antibody is relative to monoclonal antibody, which usually comprises at least two or more different antibodies, and these different antibodies usually recognize different epitopes on an antigen. Monoclonal antibodies can usually be obtained using the hybridoma technology that was first reported by Kohler et al. (Köhler G, Milstein C. Continuous cultures of fused cells secreting antibody of predefined specificity[J]. nature, 1975; 256(5517): 495), but also can be obtained using the recombinant DNA technology (e.g., see, U.S. Patent 4,816,567).

The term "single-chain antibody (single chain fragment variable, ScFv)" refers to a molecule comprising an antibody heavy chain variable region (V_{H}) and an antibody light chain variable region (V_{L}) connected by a linker, wherein the V_{L} and V_{H} domains are paired to form a monovalent molecule via a linker that enables it to produce single polypeptide chains (see, for example, Bird et al, Science 1988; 242: 423 426 and Huston et al, Proc. Natl. Acad. Sci. USA 1988; 85: 5879 5883). Such scFv molecules may have the general structure of: NH₂-V_{L}-linker-V_{H}-COOH or NH₂-V_{H}-linker-V_{L}-COOH. A suitable prior art linker may consist of repeated GGGGS amino acid sequences or variants thereof. For example, a linker having the amino acid sequence (GGGGS)₄ (SEQ ID NO: 23) can be used, but variants thereof can also be used (Holliger et al, Proc. Natl. Acad. Sci. USA 1993; 90: 6444-6448). Other linkers useful in the present invention are described by Alfthan et al, Protein Eng. 1995; 8: 725-731, Choi et al, Eur. J. Immunol. 2001; 31: 94-106, Hu et al, Cancer Res. 1996; 56: 3055-3061, Kipriyanov et al, J. Mol. Biol. 1999; 293: 41-56, and Roovers et al, Cancer Immunology, Immunotherapy, 2001, 50(1): 51-59. Description.

As used herein, the term "isolated" or "being isolated" refers to being obtained from the natural state by artificial means. If an "isolated" substance or ingredient occurs in nature, it may be due to a change in its natural environment, or the substance has been separated from its natural environment, or both. For example, a certain unisolated polynucleotide or polypeptide naturally exists in a living animal, and the same polynucleotide or polypeptide with high purity isolated from this natural state is called "isolated", of. The term "isolated" or "being isolated" does not exclude the admixture of artificial or synthetic substances, nor does it exclude the presence of other impure substances that do not affect the activity of the substance.

As used herein, the term "vector" refers to a nucleic acid delivery vehicle into which a polynucleotide can be inserted. When a vector can express the protein encoded by the inserted polynucleotide, the vector is called an expression vector. The vector can be introduced into a host cell through transformation, transduction or transfection, so that the genetic material elements it carries can be expressed in the host cell. Vectors are well known to those skilled in the art, including but not limited to: plasmids; phagemids; cosmids; artificial chromosomes, such as yeast artificial chromosomes (YAC), bacterial artificial chromosomes (BAC) or P1-derived artificial chromosomes (PAC); phages such as λ phage or M13 phage, and animal viruses, etc. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papillomavirus, papovavirus (e.g., SV40). A vector may comprise a variety of expression-controlling elements, including, but not limited to, promoter sequence, transcription initiation sequence, enhancer sequence, selection element, and reporter gene. In addition, the vector may also comprise an origin of replication site.

As used herein, the term "host cell" refers to a cell that can be used to introduce a vector, which includes, but is not limited to, prokaryotic cell such as *Escherichia coli* or *Bacillus subtilis,* fungal cell such as yeast cell or *Aspergillus,* etc., insect cell such as *S2 Drosophila* cell or Sf9, or animal cell such as fibroblast, CHO cell, GS cell, COS cell, NSO cell, HeLa cell, BHK cell, HEK 293 cell or human cell.

As used herein, the term "specific binding" refers to a non-random binding reaction between two molecules, such as the reaction between an antibody and an antigen to which it targets. In certain embodiments, an antibody that specifically binds to an antigen (or an antibody specific for an antigen) refers to an antibody that binds to the antigen with an affinity (K_{D}) of less than about 10⁻⁵ M, such as less than about 10⁻⁶ M, 10⁻⁷ M, 10⁻⁸ M, 10⁻⁹ M, or 10⁻¹⁰ M or less.

As used herein, the term "K_{D}" refers to the dissociation equilibrium constant of a specific antibody-antigen interaction, which is used to describe the binding affinity between the antibody and the antigen. The smaller the equilibrium dissociation constant, the tighter the antibody-antigen binding, and the higher the affinity between the antibody and the antigen. Typically, an antibody binds to an antigen (e.g., TIGIT protein or PD-L1 protein) with a dissociation equilibrium constant (K_{D}) of less than about 10⁻⁵ M, such as less than about 10⁻⁶ M, 10⁻⁷ M, 10⁻⁸ M, 10⁻⁹ M, or 10⁻¹⁰ M or less. K_{D} can be determined using methods known to those skilled in the art, such as using a Fortebio molecular interaction instrument.

As used herein, the terms "monoclonal antibody" and "McAb" have the same meaning and are used interchangeably; the terms "polyclonal antibody" and "PcAb" have the same meaning and are used interchangeably. And in the present invention, amino acids are generally represented by one-letter and three-letter abbreviations well known in the art. For example, alanine can be represented by A or Ala.

As used herein, the term "pharmaceutically acceptable excipient" refers to a carrier and/or excipient that is pharmacologically and/or physiologically compatible with a subject and an active ingredient. It is well known in the art (see, for example, Remington's Pharmaceutical Sciences. Edited by Gennaro AR, 19th ed. Pennsylvania: Mack Publishing Company, 1995), and includes, but is not limited to: pH adjuster, surfactant, adjuvant, ionic strength enhancer. For example, the pH adjuster includes but is not limited to phosphate buffer; the surfactant includes but is not limited to cationic, anionic or nonionic surfactant such as Tween-80; and the ionic strength enhancer includes but is not limited to sodium chloride.

As used herein, the term "effective amount" refers to an amount sufficient to obtain, at least partially, the desired effect. For example, a prophylactically effective amount is an amount that is sufficient to prevent, arrest, or delay the occurrence of a disease (e.g., a tumor); a therapeutically effective amount is an amount that is sufficient to cure or at least partially prevent a disease and its complications in a patient who already has the disease. Determining such effective amounts is well within the capabilities of those skilled in the art. For example, the amount effective for therapeutic use will depend on the severity of the disease to be treated, the overall status of the patient's own immune system, the patient's general condition such as age, weight and gender, the manner in which the drug is administered, and other treatments administered concurrently etc.

As used herein, when referring to the amino acid sequence of PD-L1 protein (Programmed death-ligand 1, NCBI GenBank ID: NP_054862.1), it comprises the full length of human PD-L1 protein, or human PD-L1 extracellular fragment PD-L1 ECD (e.g., the amino acid sequence as set forth in SEQ ID NO: 6) or a fragment containing PD-L1 ECD; and it also comprises a fusion protein of PD-L1 ECD, such as a fragment fused to a mouse or human IgG Fc protein fragment (mFc or hFc). However, those skilled in the art understand that in the amino acid sequence of PD-L1 protein, a mutation or variation (including but not limited to substitution, deletion and/or addition) can occur naturally or be artificially introduced without affecting its biological function. Therefore, in the present invention, the term "PD-L1 protein" shall include all such sequences, including the sequences shown and natural or artificial variants thereof. Moreover, when describing a sequence fragment of PD-L1 protein, it includes not only the sequence fragment, but also the corresponding sequence fragment in its natural or artificial variants.

As used herein, when referring to the amino acid sequence of TIGIT protein (T cell immunoreceptor with Ig and ITIM domains, NCBI GenBank ID: NP_776160.2), it comprises the full length of human TIGIT protein, or the extracellular fragment TIGIT ECD of human TIGIT (e.g., the amino acid sequence as set forth in SEQ ID NO: 8) or a fragment containing TIGIT ECD; and it also comprises a full-length fusion protein of TIGIT protein or a fusion protein of TIGIT ECD, such as a fragment fused to a Fc protein fragment (mFc or hFc) of mouse or human IgG. However, those skilled in the art understand that in the amino acid sequence of the TIGIT protein, a mutation or variation (including but not limited to substitution, deletion and/or addition) can occur naturally or be artificially introduced without affecting its biological function. Therefore, in the present invention, the term "TIGIT protein" shall include all such sequences, including natural or artificial variants thereof. Furthermore, when describing a sequence fragment of TIGIT protein, it also includes the corresponding sequence fragment in its natural or artificial variants.

As used herein, when referring to the amino acid sequence of PD-1 protein (NCBI GenBank: NP_005009.2), it comprises the full length of human PD-1 protein, or the extracellular fragment PD-1ECD of human PD-1, or a fragment containing PD-1 ECD; and it also comprises the full-length of a fusion protein of PD-1 protein or a fusion protein of PD-1 ECD, such as a fragment fused to a Fc protein fragment (mFc or hFc) of mouse or human IgG. However, those skilled in the art understand that in the amino acid sequence of PD-1 protein, a mutation or variation (including but not limited to substitution, deletion and/or addition) can occur naturally or be artificially introduced without affecting its biological function. Therefore, in the present invention, the term "PD-1 protein" shall include all such sequences, including natural or artificial variants thereof. And, when describing a sequence fragment of PD-1 protein, it also includes the corresponding sequence fragment in its natural or artificial variants.

In the present invention, the terms "single-domain antibody", "VHH" and "nanobody" have the same meaning and refer to cloning a variable region of heavy chain of an antibody and constructing a nanobody (VHH) consisting of only one heavy chain variable region, which is the smallest fully functional antigen-binding fragment. Usually, after obtaining an antibody that naturally lacks the light chain and heavy chain constant region 1 (CH1), the variable region of the antibody heavy chain is cloned to construct a nanobody (VHH) consisting of only one heavy chain variable region.

In the present invention, unless otherwise stated, the terms "first" (e.g., first protein functional region, first peptide chain) and "second" (e.g., second protein functional region, second peptide chain) are used for reference distinction or clarity, and do not have typical sequential meaning.

In the present invention, the single-end antibody molecule, unless otherwise specified, refers to an antibody molecule that is the same as or similar to the first protein functional region molecule or the second protein functional region molecule in the bispecific antibody, for example, an anti-TIGIT monoclonal antibody, anti-PD-L1 monoclonal antibody, or anti-PD-L1 single-domain antibody that is the same as or similar to the first protein functional region molecule or the second protein functional region molecule in the bispecific antibody.

### Beneficial effects of the present invention

The present invention achieves one or more of the following effects:
(1) the anti-TIGIT antibody of the present invention has superior affinity and specificity;
(2) the bispecific antibody of the present invention can specifically bind to TIGIT very well;
(3) the bispecific antibody of the present invention can specifically bind to PD-L1 very well;
(4) the bispecific antibody of the present invention can simultaneously bind to human TIGIT and human PD-L1 protein;
(5) there is a synergistic effect between the first protein functional region and the second protein functional region in the bispecific antibody of the present invention. For example: the anti-TIGIT/anti-PD-L1 bispecific antibody of the present invention can respectively relieve the CD155/CD112-mediated TIGIT downstream inhibitory signaling pathway and the PD-L1-mediated PD-1 downstream inhibitory signaling pathway, and its activity is better than that of the combination of two single-end antibody molecules; and/or its anti-tumor activity in vivo is better than that of the combination of two single-end antibody molecules.
(6) the bispecific antibody of the present invention is easy to produce;
(7) the bispecific antibody of the present invention has good stability and long half-life.

### Brief Description of the Drawings

Figure 1 shows the schematic structural diagram of the bispecific antibody of the present invention.
Figure 2A shows the binding curve of the bispecific antibody of the present invention to human PD-L1 overexpressed on CHO cells.
Figure 2B shows the binding curve of the bispecific antibody of the present invention to cynomolgus monkey PD-L1 overexpressed on CHO cells.
Figure 2C shows the curve that the bispecific antibody of the present invention blocks the binding of human PD-L1 to human PD-1 overexpressed on CHO cells.
Figure 3A shows the binding curve of the bispecific antibody of the present invention to human TIGIT overexpressed on CHO cells.
Figure 3B shows the binding curve of the bispecific antibody of the present invention to cynomolgus monkey TIGIT overexpressed on CHO cells.
Figure 3C shows the binding curve of the bispecific antibody of the present invention to mouse TIGIT overexpressed on CHO cells.
Figure 3D shows the curve that the bispecific antibody of the present invention blocks the binding of human CD155 to human TIGIT overexpressed on CHO cells.
Figure 3E shows the curve that the bispecific antibody of the present invention blocks the binding of mouse CD155 to mouse TIGIT overexpressed on CHO cells.
Figure 4 shows the curve that the bispecific antibody of the present invention simultaneously binds to human PD-L1 and human TIGIT protein.
Figure 5 shows the curve that the bispecific antibody of the present invention blocks the PD-1/PD-L1 and TIGIT/CD155/CD112 signaling pathways.
Figure 6A and Figure 6B show the statistical graph of the amount of cytokines release in the mixed lymphocyte assay using the bispecific antibody of the present invention. Wherein, the PBMC cell samples used in Figure 6A and Figure 6B were from different donors.
Figure 7 shows the pharmacodynamic curve of the bispecific antibody of the present invention in the B-NDG mouse model co-inoculated with A375 and human PBMC.
Figure 8 shows the dose-dependent pharmacodynamic curve of the bispecific antibody of the present invention in the B-NDG mouse model co-inoculated with A375 and human PBMC.
Figure 9 shows the pharmacodynamic curve of the bispecific antibody of the present invention in the human PD-L1/PD-1/TIGIT transgenic mouse CT26 tumor model.
Figure 10 shows the half-life curve of the bispecific antibody of the present invention in mice.
Figure 11 shows the binding curve of the anti-TIGIT antibody of the present invention to human TIGIT overexpressed on CHO cells.
Figure 12 shows the binding curve of the anti-TIGIT antibody of the present invention to cynomolgus monkey TIGIT overexpressed on CHO cells.
Figure 13 shows the binding curve of the anti-TIGIT antibody of the present invention to mouse TIGIT overexpressed on CHO cells.
Figure 14 shows the curve that the anti-TIGIT antibody of the present invention blocks the binding of human CD155 to human TIGIT overexpressed on CHO cells.
Figure 15 shows the curve that the anti-TIGIT antibody of the present invention blocks the binding of mouse CD155 to mouse TIGIT overexpressed on CHO cells.
Figure 16 shows the binding curve of the anti-TIGIT antibody of the present invention to the TIGIT on the activated human primary T cells.

The sequences involved in the present invention are shown in Table A below, in which the CDRs are each determined according to the Kabat numbering system.

**Table A**

| Name or meaning | Sequence | SEQ ID NO: |
|---|---|---|
| Anti-TIGIT intact wild-type IgG1 antibody heavy chain | QLQLQESGPGLVKPAETLSLTCTVSGGSISSYD HYWTWIRQPPGKGLEWIGTVYYSGSTFHNPSL KSRVTIPVDTSKNQFSLKLSSVTAADTAVYYC ARVGPDVSHPPFDYWGQGTLVTVSSASTKGPS VFPLAPSSKSTSGGTAALGCLVKDYFPEPVTVS WNSGALTSGVHTFPAVLQSSGLYSLSSVVTVP SSSLGTQTYICNVNHKPSNTKVDKKVEPKSCD KTHTCPPCPAPELLGGPSVFLFPPKPKDTLMIS RTPEVTCVVVDVSHEDPEVKFNWYVDGVEVH NAKTKPREEQYNSTYRVVSVLTVLHQDWLNG KEYKCKVSNKALPAPIEKTISKAKGQPREPQV YTLPPSRDELTKNQVSLTCLVKGFYPSDIAVE WESNGQPENNYKTTPPVLDSDGSFFLYSKLTV DKSRWQQGNVFSCSVMHEALHNHYTQKSLSL SPG | 1 |
| Linker G₄S | GGGGSGGGGS | 2 |
| Anti-PD-L1 single-domain antibody (VHH) | EVQLQESGGGLVQPGGSLRLSCAASGFTFSSY WMYWLRQAPGKGLEWVSSINSDSSSTYYRDS VKGRFTISRDNAKNTLYLQMNSLKSEDTAVY YCAKDPGGYAKGQGTQVTVSS | 3 |
| Anti-TIGIT/anti-PD-L1 bispecific antibody peptide chain #1 | QLQLQESGPGLVKPAETLSLTCTVSGGSISSYD HYWTWIRQPPGKGLEWIGTVYYSGSTFHNPSL KSRVTIPVDTSKNQFSLKLSSVTAADTAVYYC ARVGPDVSHPPFDYWGQGTLVTVSSASTKGPS VFPLAPSSKSTSGGTAALGCLVKDYFPEPVTVS WNSGALTSGVHTFPAVLQSSGLYSLSSVVTVP SSSLGTQTYICNVNHKPSNTKVDKKVEPKSCD KTHTCPPCPAPELLGGPSVFLFPPKPKDTLMIS RTPEVTCVVVDVSHEDPEVKFNWYVDGVEVH NAKTKPREEQYNSTYRVVSVLTVLHQDWLNG KEYKCKVSNKALPAPIEKTISKAKGQPREPQV YTLPPSRDELTKNQVSLTCLVKGFYPSDIAVE WESNGQPENNYKTTPPVLDSDGSFFLYSKLTV DKSRWQQGNVFSCSVMHEALHNHYTQKSLSL SPGGGGGSGGGGSEVQLQESGGGLVQPGGSL RLSCAASGFTFSSYWMYWLRQAPGKGLEWVS SINSDSSSTYYRDSVKGRFTISRDNAKNTLYLQ MNSLKSEDTAVYYCAKDPGGYAKGQGTQVT VSS | 4 |
| Anti-TIGIT/anti-PD-L1 bispecific antibody peptide chain #2 (anti-TIGIT kappa light chain) | DIQMTQSPSSLSASVGDRVTITCRASQSISSYLN WYQQKPGKAPKLLIYAASSLQSGVPSRFSGSG SGTDFTLTISSLQPEDFATYYCQQSYSTPITFGG GTKVEIKRTVAAPSVFIFPPSDEQLKSGTASVV CLLNNFYPREAKVQWKVDNALQSGNSQESVT EQDSKDSTYSLSSTLTLSKADYEKHKVYACEV THQGLSSPVTKSFNRGEC | 5 |
| Human PD-L1 extracellular fragment amino acid sequence | FTVTVPKDLYVVEYGSNMTIECKFPVEKQLDL AALIVYWEMEDKNIIQFVHGEEDLKVQHSSYR QRARLLKDQLSLGNAALQITDVKLQDAGVYR CMISYGGADYKRITVKVNAPYNKINQRILVVD PVTSEHELTCQAEGYPKAEVIWTSSDHQVLSG KTTTTNSKREEKLFNVTSTLRINTTTNEIFYCTF RRLDPEENHTAELVIPELPLAHPPNER | 6 |
| Cynomolgus monkey PD-L1 extracellular fragment amino acid sequence | FTVTVPKDLYVVEYGSNMTIECKFPVEKQLDL TSLIVYWEMEDKNIIQFVHGEEDLKVQHSNYR QRAQLLKDQLSLGNAALRITDVKLQDAGVYR CMISYGGADYKRITVKVNAPYNKINQRILVVD PVTSEHELTCQAEGYPKAEVIWTSSDHQVLSG KTTTTNSKREEKLLNVTSTLRINTTANEIFYCIF RRLDPEENHTAELVIPELPLALPPNERT | 7 |
| Human TIGIT extracellular fragment amino acid sequence | MMTGTIETTGNISAEKGGSIILQCHLSSTTAQV TQVNWEQQDQLLAICNADLGWHISPSFKDRV APGPGLGLTLQSLTVNDTGEYFCIYHTYPDGT YTGRIFLEVLESSVAEHGARFQIP | 8 |
| Cynomolgus monkey TIGIT extracellular fragment amino acid sequence | MMTGTIETTGNISAKKGGSVILQCHLSSTMAQ VTQVNWEQHDHSLLAIRNAELGWHIYPAFKD RVAPGPGLGLTLQSLTMNDTGEYFCTYHTYPD GTYRGRIFLEVLESSVAEHSARFQI | 9 |
| HCDR1 of anti-TIGIT intact wild-type IgG1 heavy chain antibody | SYDHYWT | 10 |
| HCDR2 of anti-TIGIT intact wild-type IgG1 heavy chain antibody | TVYYSGSTFHNPSLKS | 11 |
| HCDR3 of anti-TIGIT intact wild-type IgG1 heavy chain antibody | VGPDVSHPPFDY | 12 |
| LCDR1 of anti-TIGIT kappa light chain | RASQSISSYLN | 13 |
| LCDR2 of anti-TIGIT kappa light chain | AASSLQS | 14 |
| LCDR3 of anti-TIGIT kappa light chain | QQSYSTPIT | 15 |
| HCDR1 of anti-PD-L1 VHH | SYWMY | 16 |
| HCDR2 of anti-PD-L1 VHH | SINSDSSSTYYRDSVKG | 17 |
| HCDR3 of anti-PD-L1 VHH | DPGGYA | 18 |
| Anti-TIGIT intact wild-type IgG1 heavy chain variable region (VH) | QLQLQESGPGLVKPAETLSLTCTVSGGSISSYD HYWTWIRQPPGKGLEWIGTVYYSGSTFHNPSL KSRVTIPVDTSKNQFSLKLSSVTAADTAVYYC ARVGPDVSHPPFDYWGQGTLVTVSS | 19 |
| Anti-TIGIT kappa light chain variable region (VL) | DIQMTQSPSSLSASVGDRVTITCRASQSISSYLN WYQQKPGKAPKLLIYAASSLQSGVPSRFSGSG SGTDFTLTISSLQPEDFATYYCQQSYSTPITFGG GTKVEIK | 20 |
| Mouse TIGIT extracellular fragment amino acid sequence | TIDTKRNISAEEGGSVILQCHFSSDTAEVTQVD WKQQDQLLAIYSVDLGWHVASVFSDRVVPGP SLGLTFQSLTMNDTGEYFCTYHTYPGGIYKGR IFLKVQESSDDRNGLAQFQTAPLG | 21 |

### Specific Models for Carrying Out the present invention

The present invention will be further described below in conjunction with specific examples. It should be understood that these examples are only used to illustrate the present invention and are not intended to limit the scope of the present invention. The experimental methods without specifying specific conditions in the following examples usually followed conventional conditions, such as the conditions described in Sambrook et al., Molecular Cloning: Laboratory Manual (New York: Cold Spring Harbor Laboratory Press, 1989), or the conditions recommended by the manufacturer. Unless otherwise stated, percentages and parts are by weight.
Control antibody Atezolizumab: anti-PD-L1 monoclonal antibody, trade name: Tecentriq, Roche.
Control antibody Tiragolumab: anti-TIGIT monoclonal antibody, Roche.

### Example 1: Expression and purification of anti-TIGIT/anti-PD-L1 bispecific antibody

In this example, the anti-TIGIT antibody heavy chain variable region (VH) sequence (SEQ ID NO: 19) was synthesized using full gene synthesis and recombined into the wild-type hIgG 1 antibody heavy chain to form a complete "VH-CH1-CH2-CH3" antibody heavy chain (SEQ ID NO: 1), and anti-PD-L1 VHH (SEQ ID NO: 3) was linked at its heavy chain C-terminal through 2 linkers G₄S (SEQ ID NO: 2), to obtain the full-length amino acid sequence of peptide chain #1 as set forth in SEQ ID NO: 4. In addition, the anti-TIGIT light chain variable region (VL) sequence (SEQ ID NO: 20) was synthesized using full gene synthesis and recombined into the kappa light chain to form a complete "VL-CL" antibody light chain structure. The full-length amino acid sequence of the obtained peptide chain #2 was set forth in SEQ ID NO: 5.

Molecular cloning technology was used to construct the heavy chain and light chain sequences into pcDNA3.1 expression frame respectively, and conventional expression was carried out through Expi-293 expression system. The transfection method was performed according to the product instructions, in which the supernatant was collected after 5 days of cell culture, and the target protein was purified using protein A magnetic beads (purchased from GenScript). The magnetic beads were resuspended in an appropriate volume of a binding buffer (PBS+0.1% Tween 20, pH 7.4) (1 to 4 times the volume of the magnetic beads), then added to the sample to be purified, and incubated at room temperature for 1 hour, shaking gently during the period. The sample was placed on a magnetic stand (purchased from Beaver), the supernatant was discarded, and the magnetic beads were washed three times with the binding buffer. Elution buffer (0.1M sodium citrate, pH 3.2) with 3 to 5 times the volume of magnetic beads was added, shaken at room temperature for 5 to 10 minutes, and placed back on the magnetic stand, and the elution buffer was collected, transferred to a collection tube in which neutralization buffer (1M Tris, pH 8.54) had been added, and mixed well.

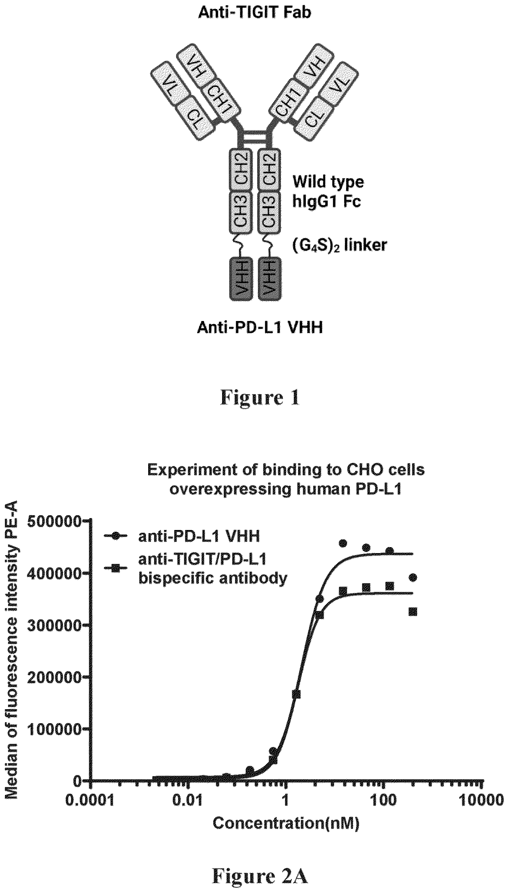

The anti-TIGIT/anti-PD-L1 bispecific antibody (also called anti-TIGIT/PD-L1 bispecific antibody in the present invention) was obtained, and its schematic structure was shown in Figure 1.

### Example 2: Detection of antibody affinity

Biofilm layer optical interference technology (ForteBio) was used to determine the binding dissociation constants (K_{D}) that the bispecific antibody obtained in Example 1 and its corresponding single-end antibody molecules bound to human and cynomolgus PD-L1 and TIGIT. Fortebio affinity measurement was carried out according to existing methods (Este, P et al. High throughput solution-based measurement of antibody-antigen affinity and epitope binning. Mabs, 2013.5(2): p.270-8), in which the human PD-L1 extracellular fragment amino acid sequence, the cynomolgus monkey PD-L1 extracellular fragment amino acid sequence, the human TIGIT extracellular fragment amino acid sequence, and the cynomolgus monkey TIGIT extracellular fragment amino acid sequence were set forth in SEQ ID NOs: 6-9, respectively.

The detailed procedure was as follows: the sensor was equilibrated offline in analysis buffer for 30 minutes, then detected online for 60 s to establish a baseline, and the purified intact antibody was loaded onto the AHQ sensor to a thickness of 1 nm for affinity detection. The antibody-loaded sensor was exposed to 100 nM human or cynomolgus monkey PD-L1, TIGIT-his antigen until the plateau phase, and then the sensor was transferred to analysis buffer for at least 2 minutes for measuring dissociation rate. Kinetic analysis was performed using a 1:1 binding model.

The K_{D} values of the bispecific antibody and its corresponding single- terminal antibody molecules in binding to human, cynomolgus monkey PD-L1 and TIGIT were shown in Table 1 below.

**Table 1: K_{D} values of anti-TIGIT/anti-PD-L1 bispecific antibody and corresponding single-terminal antibodies**

| Antibody | Human PD-L1-his (K_{D}) | Cynomolgus monkey PD-L1-his (K_{D}) | Human TIGIT-his (K_{D}) | Cynomolgus monkey TIGIT-his (K_{D}) |
|---|---|---|---|---|
| Anti-TIGIT/PD-L 1 bispecific antibody | 4.20E-09 | 3.40E-09 | 1.50E-09 | 1.60E-08 |
| Anti-PD-L1 VHH | 5.20E-09 | 5.30E-09 | N.B. | N.B. |
| Anti-TIGIT mAb | N.B. | N.B. | 1.40E-09 | 1.30E-08 |

| | | | | |
|---|---|---|---|---|
| Note: "N.B.": no binding. | | | | |

The results showed that the monovalent affinity values of the anti-TIGIT/PD-L1 bispecific antibody to human PD-L1, cynomolgus monkey PD-L1, human TIGIT, and cynomolgus monkey TIGIT antigens were comparable to or even better than the monovalent affinity values of the single-terminal antibody molecules to human PD-L1, cynomolgus monkey PD-L1, human TIGIT, and cynomolgus monkey TIGIT antigens, respectively.

### Example 3: Binding activity and blocking activity of anti-TIGIT/anti-PD-L1 bispecific antibody to CHO cells overexpressing human/cynomolgus PD-L1

3.1 Detection of binding activity of anti-TIGIT/anti-PD-L1 bispecific antibody to human/cynomolgus monkey PD-L1 overexpressed on CHO cells based on flow cytometry detection method

Specifically, CHO-S cells overexpressing human PD-L1 (CHO-huPD- L1 cells) and CHO-S cells overexpressing cynomolgus monkey PD-L1 (CHO-cynoPD-L1 cells) were generated by pressure screening by transfecting the pCHO1.0 vectors (purchased from Invitrogen) of human PD-L1 and cynomolgus monkey PD-L1 cloned into MCS. The overexpressing cells after the expanded culture were adjusted to an appropriate cell density, and added to a 96-well flow cytometry plate, then centrifuged, added with the gradiently diluted sample to be tested, and incubated at 4°C for 30 minutes; then washed twice with PBS, added with a fluorescent secondary antibody correspondingly diluted to appropriate concentration, and incubated at 4°C for 30 minutes; then washed twice with PBS, added with the cells resuspended in PBS, and detected on a CytoFlex flow cytometer, and corresponding MFI was calculated. Graphpad software was used for graphing analysis to obtain EC₅₀ values.

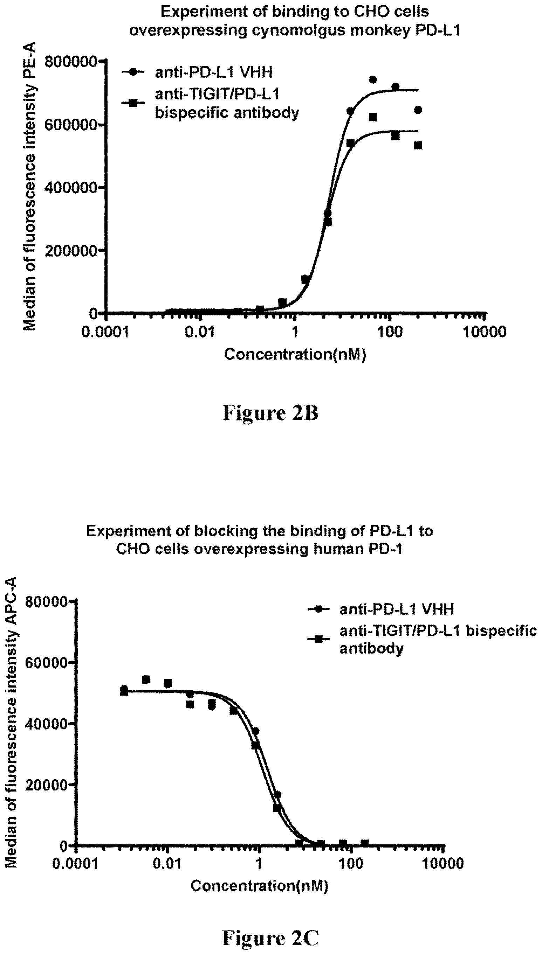

The results were shown in Table 2, Figure 2A and Figure 2B.

**Table 2: Binding activity and blocking activity of anti-TIGIT/anti-PD-L1 bispecific antibody to cells overexpressing PD-L1/PD-1**

| Antibody | EC₅₀ of binding to CHO cells overexpressing human PD-L1 (nM) | EC₅₀ of binding to CHO cells overexpressing cynomolgus monkey PD-L1 (nM) | IC₅₀ of blocking the binding of human PD-L1 to CHO cells overexpressing human PD-L1 (nM) |
|---|---|---|---|
| Anti-TIGIT/PD-L 1 bispecific antibody | 1.78 | 4.60 | 1.17 |
| Anti-PD-L1 VHH | 2.14 | 5.24 | 1.51 |

The results showed that the anti-TIGIT/anti-PD-L1 bispecific antibody of the present invention had binding activity to human/cynomolgus monkey PD-L1 overexpressed on CHO cells that was comparable to that of its PD-L1-terminal monoclonal antibody molecule (anti-PD-L1 VHH).

### 3.2 Detection of blocking activity of anti-TIGIT/anti-PD-L1 bispecific antibody on binding of human PD-L1 to human PD-1 overexpressed on CHO cells based on flow cytometry detection

Specifically, CHO-S cells overexpressing human PD-1 (CHO-huPD-1 cells) were generated by pressure screening by transfecting the pCHO1.0 vector (purchased from Invitrogen) of human PD-1 cloned into MCS. The purified antibody to be tested was diluted with PBS, the diluted sample was added into a 96-well flow cytometry plate, 60 µL/well. Then, biotinylated human PD-L1 protein was added at 60 µL/well to reach a final concentration of 0.5 µg/mL, mixed and incubated at 4°C for 30 minutes. The CHO-huPD-1 cells after expanded culture were adjusted to reach a cell density of 2×10⁶ cells/mL, added at 100 µL/well to a 96-well flow cytometry plate, and centrifuged, and the supernatant was discarded. The above co-incubated antibody-antigen mixture was added at 100 µL/well and incubated at 4°C for 30 minutes. After washing twice with PBS, streptomycin avidin-R-phycoerythrin conjugate (SAPE) diluted 100-fold with PBS was added at 100 µL/well, and incubated at 4°C for 30 minutes. After washing twice with PBS, the cells resuspended with PBS were added at 100 µL/well, and detected on a CytoFlex flow cytometer, and the corresponding MFI was calculated. Graphpad software was used for graphing analysis to obtain IC₅₀ value.

The results were shown in Table 2 and Figure 2C. The results showed that the blocking activity of the anti-TIGIT/anti-PD-L1 bispecific antibody of the present invention on the binding of human PD-L1 to human PD-1 overexpressed on CHO cells was comparable to or even better than that of its PD-L1 terminal monoclonal antibody molecule (anti-PD-L1 VHH).

### Example 4: Binding activity and blocking activity of anti-TIGIT/anti-PD-L1 bispecific antibody to CHO cells overexpressing human/cynomolgus monkey/mouse TIGIT

### 4.1 Detection of binding activity of anti-TIGIT/anti-PD-L1 bispecific antibody to human/cynomolgus monkey/mouse TIGIT overexpressed on CHO cells based on flow cytometry detection method

Specifically, CHO-S cells overexpressing human TIGIT (CHO-huTIGIT cells), CHO-S cells overexpressing cynomolgus monkey TIGIT (CHO-cynoTIGIT cells) and CHO-S cells overexpressing mouse TIGIT (CHO-muTIGIT cells) were generated through pressure screening by transfecting the pCHO1.0 vectors (purchased from Invitrogen) of human TIGIT, cynomolgus monkey TIGIT and mouse TIGIT cDNA cloned into MCS. The overexpressing cells after the expanded culture were adjusted to a suitable cell density and added to a 96-well flow cytometry plate, centrifuged and then added with the gradiently diluted sample to be tested, and incubated at 4°C for 30 minutes. After washing twice with PBS, fluorescent secondary antibody correspondingly diluted to an appropriate concentration was added, and incubated at 4°C for 30 minutes. After washing twice with PBS, the cells resuspended in PBS was added, and detected on a CytoFlex flow cytometer, and the corresponding MFI was calculated. Graphpad software was used for graphing analysis to obtain EC₅₀ value.

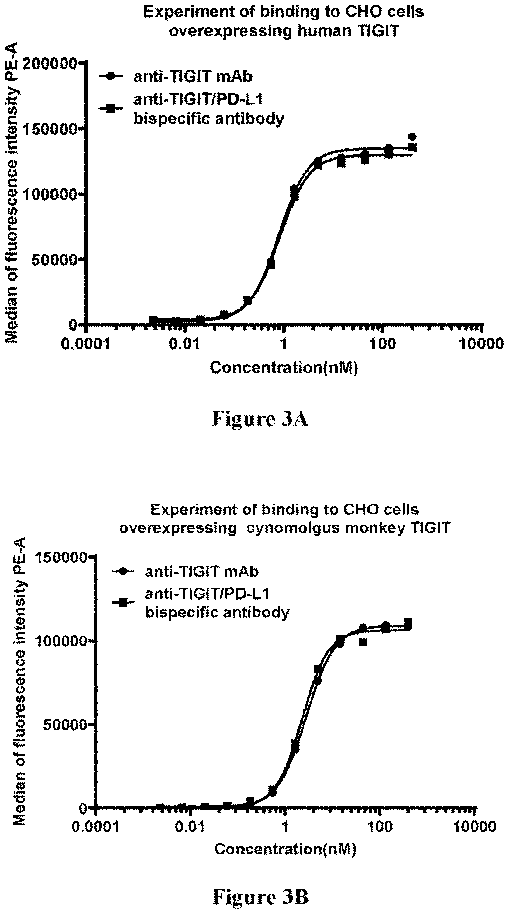

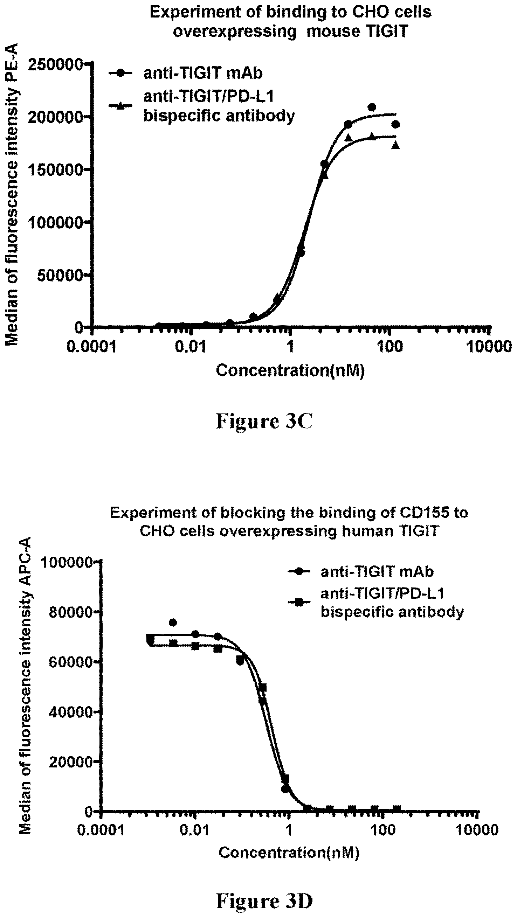

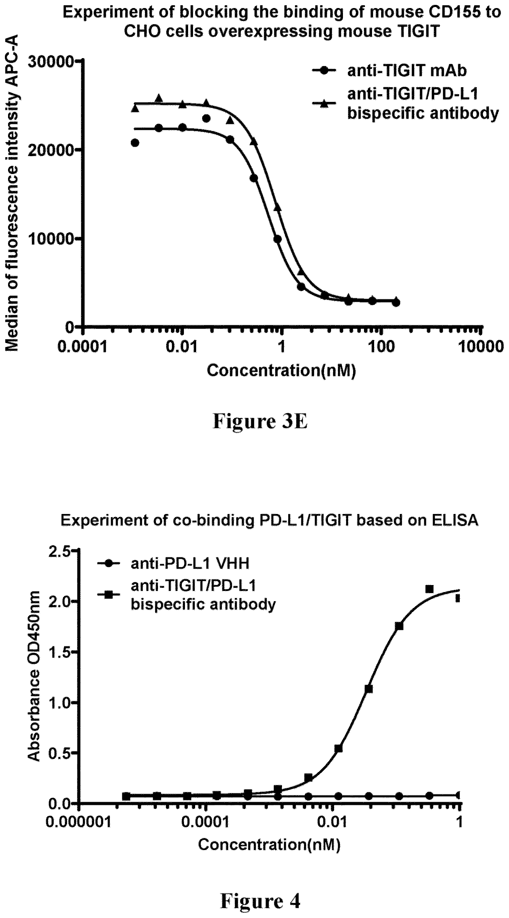

The results were shown in Table 3, and Figure 3A to Figure 3C.

**Table 3: Binding activity and blocking activity of anti-TIGIT/anti-PD-L1 bispecific antibody to cells overexpressing TIGIT**

| Antibody | EC₅₀ of binding to CHO cells overexpressing human TIGIT (nM) | EC₅₀ of binding to CHO cells overexpressing cynomolgus monkey TIGIT (nM) | EC₅₀ of binding to CHO cells overexpressing mouse TIGIT (nM) | IC₅₀ of blocking the binding of human CD155 to CHO cells overexpressing human TIGIT (nM) | IC₅₀ of blocking the binding of mouse CD155 to CHO cells overexpressing mouse TIGIT (nM) |
|---|---|---|---|---|---|
| Anti-TIGIT/PD-L1 bispecific antibody | 0.83 | 2.32 | 1.94 | 0.43 | 0.75 |
| Anti-TIGIT mAb | 0.80 | 2.80 | 2.40 | 0.33 | 0.54 |

The results showed that the binding activity of the anti-TIGIT/anti-PD-L1 bispecific antibody of the present invention to human/cynomolgus monkey/mouse TIGIT overexpressed on CHO cells was comparable to or even better than that of its TIGIT terminal monoclonal antibody molecule (anti-TIGIT mAb).

### 4.2 Detection of blocking activity of anti-TIGIT/anti-PD-L1 bispecific antibody based on flow cytometry for the binding of human CD155 to human TIGIT overexpressed on CHO cells, and the binding of mouse CD155 to mouse TIGIT overexpressed on CHO cells

Specifically, the CHO-huTIGIT cells after expanded culture were adjusted to a cell density of 2×10⁶ cells/mL, added to a 96-well flow cytometry plate, 100 µL/well, and centrifuged for later use. The purified monoclonal antibody was diluted with PBS, starting at 400 nM with 3-fold dilution for a total of 12 points. The diluted sample was added at 60 µL/well to the above-mentioned 96-well flow cytometry plate with cells, and incubated at 4°C for 30 minutes. Then human CD155 protein with Mouse IgG2a Fc Tag was added at 60 µL/well to reach a final concentration of 2 µg/mL, and incubated at 4°C for 30 minutes. After washing twice with PBS, APC goat anti-mouse IgG antibody diluted 100 times in PBS was added at 100 µL/well, and incubated at 4°C for 30 minutes. After washing twice with PBS, the cells resuspended in PBS was added at 100 µL/well, and detected on a CytoFlex flow cytometer, and the corresponding MFI was calculated.

The CHO-muTIGIT cells after expanded culture were adjusted to reach a cell density of 2×10⁶ cells/mL, added to a 96-well flow cytometry plate, 100 µL/well, and centrifuged for later use. The purified monoclonal antibody was diluted with PBS, starting at 400 nM with 3-fold dilution for a total of 12 points. The diluted sample was added at 60 µL/well to the above-mentioned 96-well flow cytometry plate with cells, and incubated at 4°C for 30 minutes. Then mouse CD155 protein with Mouse IgG2a Fc Tag was added at 60 µL/well to reach a final concentration of 2 µg/mL, and incubated at 4°C for 30 minutes. After washing twice with PBS, APC goat anti-mouse IgG antibody diluted 100 times in PBS was added at 100 µL/well, and incubated at 4°C for 30 minutes. After washing twice with PBS, the cells resuspended in PBS was added at 100 µL/well, and detected on a CytoFlex flow cytometer, and the corresponding MFI was calculated. Graphpad software was used for graphing analysis to obtain IC₅₀ value.

The results were shown in Table 3, and Figure 3D to Figure 3E. The results showed that the blocking activities of anti-TIGIT/anti-PD-L1 bispecific antibody of the present invention on the binding of human CD155 to human TIGIT overexpressed on CHO cells and on the binding of mouse CD155 to mouse TIGIT overexpressed on CHO cells were comparable to that of its TIGIT-terminal monoclonal antibody molecule (anti-TIGIT mAb).

### Example 5: Co-binding activity of anti-TIGIT/anti-PD-L1 bispecific antibody to human TIGIT and human PD-L1

The co-binding activity of the anti-TIGIT/anti-PD-L1 bispecific antibody of the present invention to human TIGIT and human PD-L1 proteins was detected based on an enzyme-linked immunosorbent assay (ELISA).

Specifically, human TIGIT protein was dissolved according to the instructions, diluted to 1 µg/mL with 1× ELISA coating solution, coated at 100 µL/well in a 96-well ELISA plate, covered with film and placed at 4°C overnight. The coating solution was discarded, washing was performed 3 times with 1×PBST, and 5% BSA/PBS was added at 200 µL/well for blocking at room temperature for 2 hours. The blocking solution was discarded, the antibody to be tested gradiently diluted in 1% BSA/PBS was added at 100 µL/well, and incubated at room temperature for 2 hours. The antibody diluent was discarded, washing was performed 3 times with 1×PBST, biotin-labeled PD-L1 protein diluted in 1% BSA/PBS was added at 100 µL/well to reach a final concentration of 1 µg/mL, and incubated at room temperature for 1 hour. The antigen diluent was discarded, washing was performed 3 times with 1×PBST, SA-HRP diluted in 1% BSA/PBS was added at 100 µL/well, and incubated at room temperature for 1 hour. The SA-HRP diluent was discarded, washing was performed 3 times with 1×PBST, ELISA chromogenic solution was added at 100 µL/well and reacted at room temperature for 1 to 3 minutes, then ELISA stop solution was added at 50 µL/well, and the absorbance value at 450 nm was read. Graphpad software was used to plot the concentration-absorbance value binding curve.

The results were shown in Figure 4. The anti-TIGIT/anti-PD-L1 bispecific antibody of the present invention could simultaneously bind to human TIGIT and human PD-L1 proteins.

### Example 6: Blocking activity of anti-TIGIT/anti-PD-L1 bispecific antibody on PD-1/PD-L1 and TIGIT/CD155/CD112 signaling pathways in luciferase reporter gene system

In order to further detect the activity of the anti-TIGIT/anti-PD-L1 bispecific antibody at the cellular level for simultaneously blocking PD-1/PD-L1 and TIGIT/CD155/CD112 signaling pathways, the following luciferase reporter gene system was constructed in this example. Briefly, lentivirus was used to transfect cells to construct a CHO-K1 cell line (CHO-K1-CD155-CD112-PD-L1) overexpressing human CD155, human CD112, human PD-L1 and OKT-3 scFv, and a Jurkat cell line (Jurkat-TIGIT-PD-1-luc) overexpressing human TIGIT, human PD-1, and NF-AT luciferase reporter gene (purchased from Promega), and this reporter gene system was subsequently used to conduct relevant experiments.

Specifically, CHO-K1-CD155-CD112-PD-L1 functional cells were obtained by digestion, adjusted to reach a desired cell density, added at 100 µL/well to a 96-well white bottom plate, and cultured for overnight adhesion. On the next day, a suspension of Jurkat-TIGIT-PD-1-luc effector cells was prepared, and the sample to be tested was gradiently diluted with reaction medium. The white bottom plate was taken out, the culture supernatant was removed by pipetting, the above diluted sample was added at 40 µL/well to the white bottom plate, the suspension of Jurkat-TIGIT-PD-1-luc effector cells was added at 40 µL/well, and incubation was performed at 37°C, 5% CO₂ in an incubator for 6 hours, during which time the Bio-Glo TM reagent was returned to room temperature. After the culture was completed, the cells were taken out and equilibrated at room temperature for 5 minutes. The Bio-Glo TM reagent was added at 80 µL/well, and a multifunctional microplate reader was used to read the fluorescence signal value.

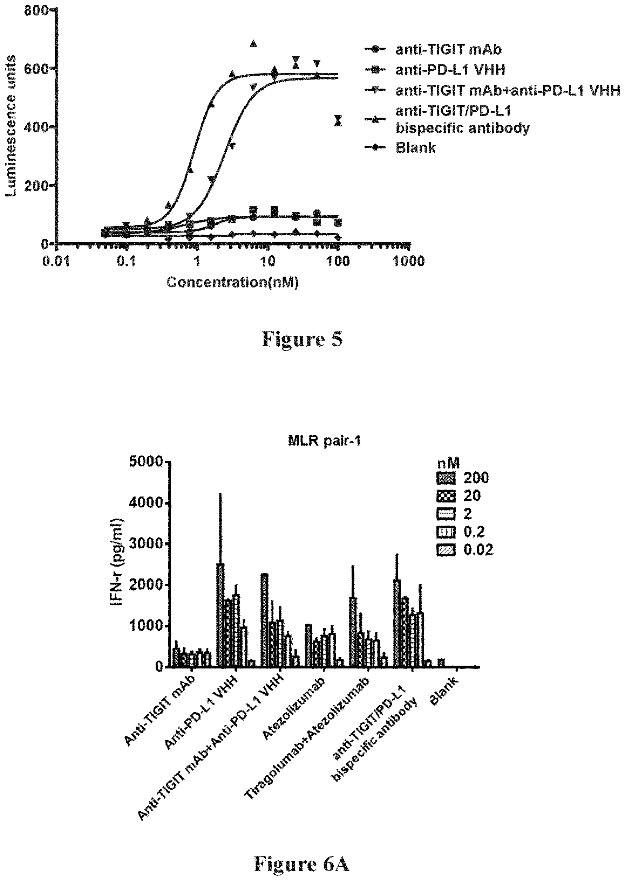

The results were shown in Figure 5. The results showed that the anti-TIGIT/anti-PD-L1 bispecific antibody of the present invention could respectively release the CD155/CD112-mediated TIGIT downstream inhibition signaling pathway and the PD-L1-mediated PD-1 downstream inhibition signaling pathway, which could up-regulate the reporter gene luciferase expression, and its activity thereof was better than that of the combination of two single- terminal antibody molecules.

### Example 7: Activity of anti-TIGIT/anti-PD-L1 bispecific antibody on T cell activation in mixed lymphocyte assay

In this example, a mixed lymphocyte reaction (MLR) assay was used to detect the activity of anti-TIGIT/anti-PD-L1 bispecific antibody in activating T cells. The detailed experimental method was as follows:
PBMC cells (purchased from SAII,YBIO, XFB-HP100B) were resuscitated and centrifuged. The PBMC cells were resuspended in 10mL of X-VIVO-15 medium (purchased from LONZA), and cultured in a cell culture incubator at 37°C for 2h, and the non-adherent cells were removed. 10mL of DC medium (X-VIVO-15 medium was supplemented with 10ng/mL GM-CSF (purchased from R&D), and 20ng/mL IL-4 (purchased from R&D)) was added, and cultured for 3 days, then 5mL of DC medium was supplemented, and the culture was continued until day 6; then DC maturation medium (X-VIVO-15 medium was added with 1000U/mL TNF-α (purchased from R&D), 10ng/mL IL-6 (purchased from R&D), 5ng/mL IL-1β (purchased from R&D), and 1 µM PGE2 (purchased from Tocris)) was added, and cultured for 2 days, then mature DC cells were collected, and the cell density was adjusted to 2×10⁵ cells/mL using X-VIVO-15 medium.

The PBMC cells from another donor (purchased from SAILY BIO, XFB-HP100B) were resuscitated, and centrifuged. The PBMC cells were resuspended in 10 mL of X-VIVO-15 medium. T cell isolation kit (purchased from Stemcell) was used to enrich T cells, X-VIVO-15 was used to resuspend the T cells, and the cell density was adjusted to 2×10⁶ cells/mL. The T cell suspension was mixed with the mature DC cells collected above at a volume ratio of 1:1, and added to a 96-well U-bottom plate at 100 µL/well.

The antibody sample to be tested was diluted with X-VIVO-15 culture medium, starting at 200 nM with 10-fold dilution for a total of 5 points, added at 100 µL/well to the above mixed cell well, and cultured for 5 days, then the supernatant was collected, and ELISA (purchased from eBioscience) method was used to detect the IFN-γ expression level.

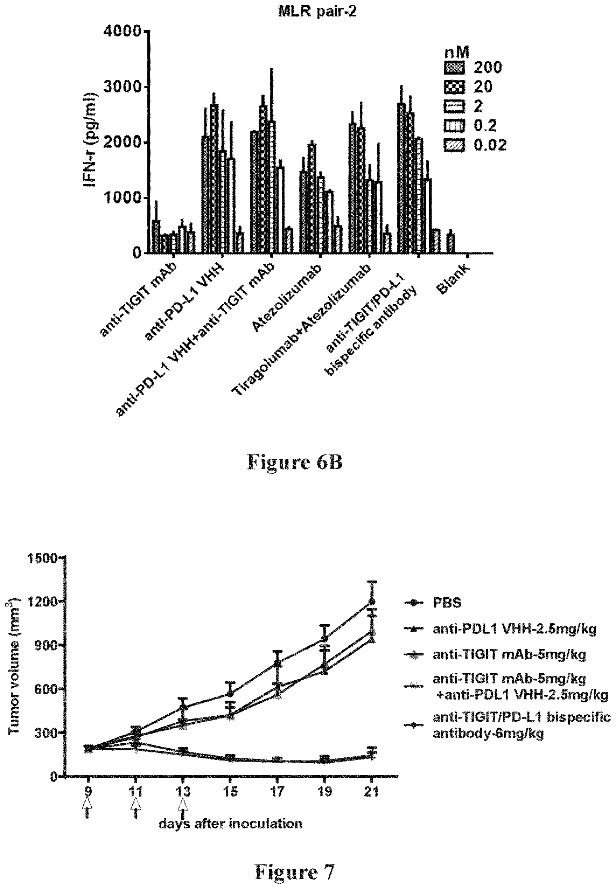

The results were shown in Figures 6A and 6B. The results showed that the anti-TIGIT/anti-PD-L1 bispecific antibody showed good biological activity in the MLR experiment, and the T cell activation level was comparable to the activity of the combination of two single- terminal antibody molecules.

### Example 8: In vivo pharmacodynamic study of anti-TIGIT/anti-PD-L1 bispecific antibody in B-NDG mice inoculated with mixed A375 and human PBMC

In this experiment, the antitumor effect of the anti-TIGIT/anti-PD-L1 bispecific antibody of the present invention was determined in a model (A375 huPBMC model) of B-NDG mice inoculated with mixed A375 (purchased from Addexbio, C0020004, a human malignant melanoma cell) and human PBMC cells (Shanghai Miles-bio, A10S033014/PB100C). Wherein, the humanized tumor mouse model that locally reconstituted the human immune system was generated by inoculating human immune cells (PBMC) into immunodeficient mice.

Specifically, A375 cells and human PBMC were first mixed at an equal volume ratio of 1:1 to obtain 0.1 mL of cell suspension, and subcutaneously injected in the right abdominal groin of mice to establish an A375 huPBMC model. Grouping was performed when the average tumor volume reached to about 200 mm³. Different doses and the same administration volume of PBS or antibody were administered intraperitoneally for treatment, with 6 mice in each group. The changes in tumor volume and body weight of mice in each group were monitored with a monitoring frequency of once per 2 to 3 days, and the monitoring was performed continuously for 2 to 3 weeks. The doses and method of administration were shown in Table 4.

**Table 4: Dosage regimen of anti-TIGIT/anti-PD-L1 bispecific antibody in A375 huPBMC model**

| Group | Dose | Dosing frequency/number of times |
|---|---|---|
| PBS | N/A | Q2d×3 |
| Anti-PD-L1 VHH | 2.5 mg/kg | Q2d×3 |
| Anti-TIGIT mAb | 5 mg/kg | Q2d×3 |
| Anti-PD-L1 VHH+Anti-TIGIT mAb | 2.5 mg/kg+5 mg/kg | Q2d×3 |
| Anti-TIGIT/PD-L1 bispecific antibody | 6 mg/kg | Q2d×3 |

| | | |
|---|---|---|
| Note: The molecular weights of anti-PD-L1 VHH, anti-TIGIT mAb, and anti-TIGIT/PD-L1 bispecific antibody were approximately 75KD, 150KD, and 175KD, respectively. The doses were based on the equimolar concentration of each component. | | |

The results were shown in Figure 7. The results showed that the anti-TIGIT/anti-PD-L1 bispecific antibody of the present invention had significant anti-tumor effect, and the effect was better than that of the combination of two single-terminal antibody molecules.

### Example 9: In vivo dose-dependent pharmacodynamic study of anti-TIGIT/anti-PD-L1 bispecific antibody in B-NDG mice inoculated with mixed A375 and human PBMC

In this experiment, the A375 huPBMC model was established by subcutaneous mixed inoculation (the steps for establishing the model were the same as in Example 8). When the average tumor volume reached to about 300 mm³, the mice were divided into groups. Different doses and the same volume of PBS or bispecific antibody were administered intraperitoneally for treatment, 6 mice in each group. The changes in tumor volume and body weight of mice in each group were monitored with a monitoring frequency of once per 2 to 3 days, and the monitoring was performed continuously for 2 to 3 weeks. The doses and method of administration were shown in Table 5.

**Table 5: Experimental protocol for dose-dependent tumor inhibitory activity of anti-TIGIT/anti-PD-L1 bispecific antibody**

| Group | Dose | Dosing frequency/number of times |
|---|---|---|
| PBS | N/A | Q3-4d×3 |
| Anti-TIGIT/PD-L1 bispecific antibody | 1.2 mg/kg | Q3-4d×3 |
| Anti-TIGIT/PD-L1 bispecific antibody | 6 mg/kg | Q3-4d×3 |
| Anti-TIGIT/PD-L1 bispecific antibody | 30 mg/kg | Q3-4d×3 |

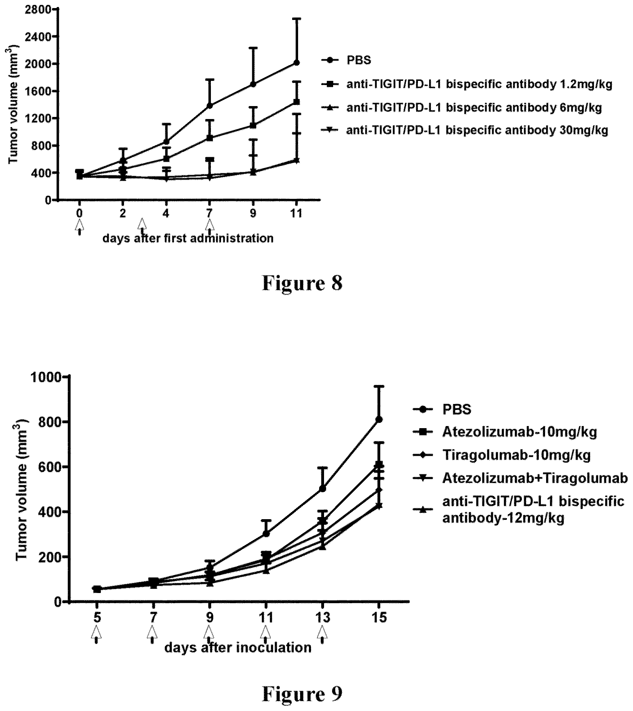

The results were shown in Figure 8. The anti-TIGIT/anti-PD-L1 bispecific antibody of the present invention could significantly inhibit the growth of mouse tumors in a dose-dependent manner.

### Example 10: In vivo pharmacodynamic study of anti-TIGIT/anti-PD-L1 bispecific antibody in huPD-L1/PD-1/TIGIT KI mice

In this experiment, the antitumor effect of the anti-TIGIT/anti-PD-L1 bispecific antibody of the present invention was determined by transplantation of CT-26-huPD-L1 tumor cells (human PD-L1 was knocked into CT26 mouse colon cancer cells, purchased from Jiangsu Gempharmatech) into human PD-L1/PD-1/TIGIT transgenic mice (huPD-L1/PD-1/TIGIT KI mice).

Specifically, CT-26-huPD-L1 cell suspension was first prepared, and 0.1 mL containing approximately 5×10⁵ cells was subcutaneously injected into the right abdominal groin of mouse to establish a CT-26-huPD-L1 tumor-bearing mouse model. When the average tumor volume reached to 80-120 mm³, the mice were divided into groups. Different doses and the same volume of PBS or antibody were administered by intraperitoneal injection for treatment, with 6 mice in each group. The changes in tumor volume and body weight of mice in each group were monitored with a monitoring frequency of once per 2 to 3 days, and the monitoring was performed continuously for 2 to 3 weeks. The doses and method of administration were shown in Table 6.

**Table 6: Dosage regimen of anti-TIGIT/anti-PD-L1 bispecific antibody in anti-huPD-L1/PD-1/TIGIT KI mouse model**

| Group | Does | Dosing frequency/number of times |
|---|---|---|
| PBS | N/A | Q2d×5 |
| Atezolizumab | 10 mg/kg | Q2d×5 |
| Tiragolumab | 10 mg/kg | Q2d×5 |
| Atezolizumab+Tiragolumab | 10mg/kg + 10mg/kg | Q2d×5 |
| Anti-TIGIT/PD-L1 bispecific antibody | 12mg/kg | Q2d×5 |

The results were shown in Figure 9. The efficacy of anti-TIGIT/anti-PD-L1 bispecific antibody of the present invention was comparable to that of the clinical drug combination (Atezolizumab + Tiragolumab), and the anti-tumor activity was superior to that of the two monoclonal antibody groups.

### Example 11: In vivo half-life study of anti-TIGIT/anti-PD-L1 bispecific antibody in mice

The tail vein single injection method was used to detect the half-life of the anti-TIGIT/anti-PD-L1 bispecific antibody of the present invention in mice.

Specifically, the experimental Balb/c mice, 3 males and 3 females, were housed in 12/12-hours of light/dark regulated environment, at a temperature of 24°C ± 2°C and a humidity of 40%-70%, and with free access to water and food. On the day of the experiment, the Balb/c mice were given a single tail vein injection of monoclonal antibody molecules at a dose of 10 mg/kg. Blood collection time points: blood was collected from the orbits of the mice at 5 minutes, 0.5 hours, 2 hours, 6 hours, 24 hours, 48 hours, 96 hours, 168 hours, 336 hours, and 504 hours after the administration. The whole blood sample were placed at 2°C to 8°C for 30 minutes, centrifuged at 12,000 rpm for 5 minutes to collect serum. The resulting serum was centrifuged at 2°C to 8°C, 12,000 rpm for 5 minutes, and stored at -80°C. The amount of the bispecific antibody molecules in the serum was detected by ELISA.

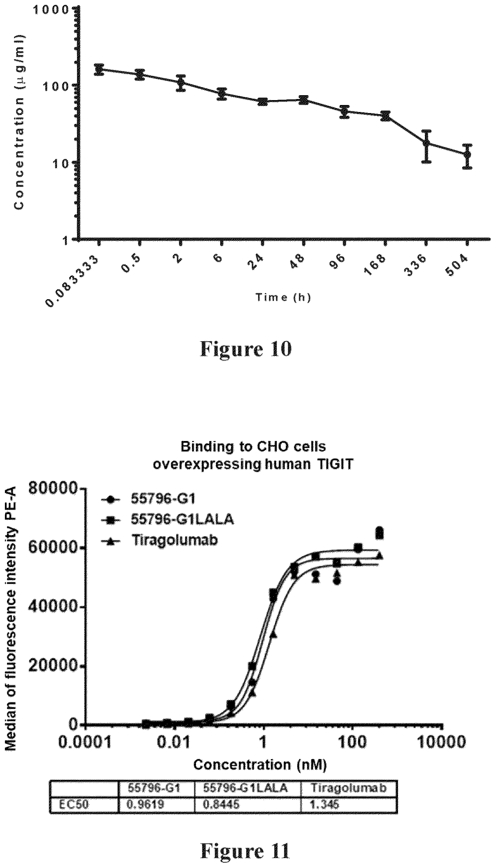

The results were shown in Figure 10. The results showed that the half-life of the anti-TIGIT/anti-PD-L1 bispecific antibody of the present invention after a single injection in mice was 189 hours.

### Preparation Example 1: Preparation of anti-TIGIT monoclonal antibody

The heavy chain variable region (the amino acid sequence was set forth in SEQ ID NO: 19) of the anti-TIGIT monoclonal antibody was recombined into the human IgG1 heavy chain constant region, as well as the human IgG1 heavy chain constant regions with L234A and L235A modifications. In addition, the light chain variable region (the amino acid sequence was set forth in SEQ ID NO: 20) was recombined into the human kappa light chain constant region, and the anti-TIGIT monoclonal antibodies were named as 55796-G1 and 55796-G1LALA, respectively. Transient expression and purification were performed via HEK293 expression system. The specific operation was as follows: the chemical transfection method was used to transfer the pcDNA3.1 vector with antibody heavy chain and light chain into HEK293 cells, and cultured at 37°C and 8% CO₂ for 7 days. The cytochylema was collected and centrifuged at 13,000 rpm for 20 minutes. The supernatant was taken, and purified with Protein A, SEC was used to detect antibody purity, and the endotoxin content was controlled at the same.

The prepared anti-TIGIT monoclonal antibodies 55796-G1 and 55796-G1LALA were used in the following Test Examples 1 to 3.

### Test Example 1: Affinity detection of anti-TIGIT monoclonal antibody

Biofilm layer optical interference technology (ForteBio) was used to determine the binding dissociation constant (K_{D}) of the anti-TIGIT monoclonal antibody prepared in Preparation Example 1 in binding human, cynomolgus monkey, and mouse TIGIT. Fortebio affinity measurement was performed according to existing method (Este, P et al. High throughput solution-based measurement of antibody-antigen affinity and epitope binning. Mabs, 2013.5(2): p.270-8). Wherein, the extracellular amino acid sequence of human TIGIT, the extracellular amino acid sequence of cynomolgus monkey TIGIT, and the extracellular amino acid sequence of mouse TIGIT were set forth in SEQ ID NOs: 8, 9 and 21, respectively.

The monovalent affinity of intact antibody (i.e., full-length IgG originally obtained from Adimab) to human, cynomolgus monkey, and mouse TIGIT-his proteins was measured: the sensor was equilibrated offline in assay buffer for 20 minutes, and then detection was performed online for 120 s to establish a baseline, the intact TIGIT antibody was loaded onto the AHQ sensor to reach a thickness of 1 nm for affinity detection. The antibody-loaded sensor was incubated in 100 nM TIGIT-his antigen until the plateau phase, and then the sensor was transferred into assay buffer for at least 2 minutes for dissociation rate measurement. Kinetic analysis was performed using a 1:1 binding model.

In the above determination method, the measured K_{D} value was shown in Table 7 below:

**Table 7: K_{D} values of anti-TIGIT monoclonal antibodies**

| Antibody | Monovalent affinity of IgG to human TIGIT-his protein | Monovalent affinity of IgG to cynomolgus monkey TIGIT-his protein | Monovalent affinity of IgG to mouse TIGIT-his protein |
|---|---|---|---|
| 55796-G1 | 1.99E-10 | 4.72E-09 | 9.60E-08 |
| 55796-G1LALA | 2.10E-10 | 4.90E-09 | 6.10E-08 |
| Tiragolumab | 8.70E-10 | 3.50E-09 | NA |

| | | | |
|---|---|---|---|
| Note: "NA": not available. | | | |

It could be seen from the results in Table 7 that: (1) the monovalent affinity of anti-TIGIT monoclonal antibody to human TIGIT-his protein was higher than that of the control molecule Tiragolumab; (2) the monovalent affinity of anti-TIGIT monoclonal antibody to cynomolgus monkey TIGIT-his protein was comparable to that of the control molecule Tiragolumab; (3) the anti-TIGIT monoclonal antibody had cross-binding activity with mouse TIGIT.

### Test Example 2: Binding activity and blocking activity of anti-TIGIT monoclonal antibody to CHO cells overexpressing human/cynomolgus monkey/mouse TIGIT

### 2.1 Detection of binding activity of anti-TIGIT monoclonal antibody to human/cynomolgus monkey/mouse TIGIT overexpressed on CHO cells based on flow cytometry

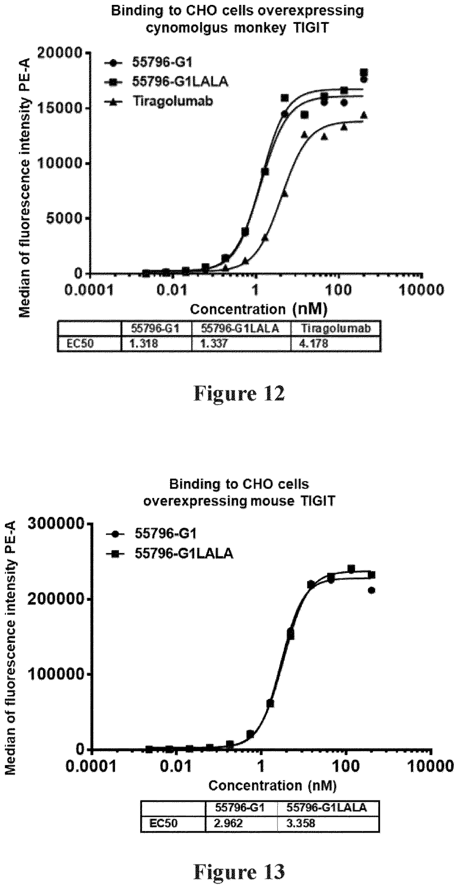

Specifically, CHO-S cells overexpressing human TIGIT (CHO-huTIGIT cells), CHO-S cells overexpressing cynomolgus monkey TIGIT (CHO-cynoTIGIT cells), and CHO-S cells overexpressing mouse TIGIT (CHO-muTIGIT cells) were generated by pressure screening by transfecting the pCHO1.0 vectors (purchased from Invitrogen) of human TIGIT, cynomolgus monkey TIGIT, and mouse TIGIT cDNAs cloned into MCS. After the expanded culture, the overexpressing cells were adjusted to an appropriate cell density and added to a 96-well flow cytometry plate. After centrifugation, the gradiently diluted samples to be tested were added, and incubated at 4°C for 30 minutes. After washing twice with PBS, fluorescent secondary antibody correspondingly diluted to an appropriate concentration was added, incubated at 4°C for 30 minutes, and washed twice with PBS. The cells resuspended in PBS were added, and detected on a CytoFlex flow cytometer, and the corresponding MFI was calculated. Graphpad software was used for graphing analysis to obtain EC₅₀ value. The results were shown in Table 8 and Figures 11 to 13.

### 2.2 Detection of blocking activity of anti-TIGIT monoclonal antibodies based on flow cytometry in blocking the binding of human CD155 to human TIGIT overexpressed on CHO cells, and in blocking the binding of mouse CD155 to mouse TIGIT overexpressed on CHO cells

Specifically, the CHO-huTIGIT cells after the expanded culture were adjusted to a cell density of 2×10⁶ cells/mL, added at 100 µL/well to a 96-well flow cytometry plate, and centrifuged for later use. The purified monoclonal antibody was diluted with PBS, starting at 400 nM with 3-fold dilution for a total of 12 points. The diluted sample was added at 60 µL/well to the above-mentioned 96-well flow cytometry plate with cells, and incubated at 4°C for 30 minutes. Then, human CD155 protein with Mouse IgG2a Fc Tag was added at 60 µL/well to reach a final concentration of 2 µg/mL, incubated at 4°C for 30 minutes, and washed twice with PBS. APC goat anti-mouse IgG antibody diluted 100 times with PBS was added at 100 µL/well, incubated at 4°C for 30 minutes, and washed twice with PBS. The cells resuspended in PBS was added at 100 µL/well, and detected on a CytoFlex flow cytometer, and the corresponding MFI was calculated.

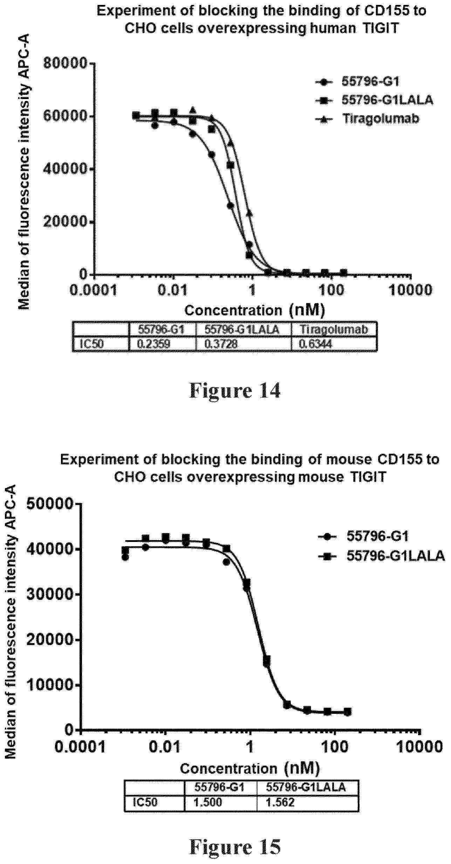

The cell density of the CHO-muTIGIT cells after the expanded culture was adjusted to 2×10⁶ cells/mL, added at 100 µL/well to a 96-well flow cytometry plate, and centrifuged for later use. The purified monoclonal antibody was diluted with PBS, starting at 400 nM with 3-fold dilution for a total of 12 points. The diluted sample was added at 60 µL/well to the above-mentioned 96-well flow cytometry plate with cells, and incubated at 4°C for 30 minutes. Then, mouse CD155 protein with Mouse IgG2a Fc Tag was added at 60 µL/well to reach a final concentration of 2 µg/mL, incubated at 4°C for 30 minutes, and washed twice with PBS. APC goat anti-mouse IgG antibody diluted 100 times with PBS was added at 100 µL/well, incubated at 4°C for 30 minutes, and washed twice with PBS. The cells resuspended in PBS was added at 100 µL/well, and detected on a CytoFlex flow cytometer, and the corresponding MFI was calculated. Graphpad software was used for graphing analysis to obtain IC₅₀ value. The results were shown in Table 8 and Figures 14 to 15.

**Table 8: Summary table of activity of anti-TIGIT monoclonal antibodies in blocking binding of overexpressing cells**

| Antibody | EC₅₀ of binding to CHO cells overexpressin g human TIGIT (nM) | EC₅₀ of binding to CHO cells overexpressing cynomolgus monkey TIGIT (nM) | EC₅₀ of binding to CHO cells overexpressin g mouse TIGIT (nM) | IC₅₀ of blocking binding of human CD155 to CHO cells overexpressing human TIGIT (nM) | IC₅₀ of blocking binding of mouse CD155 based on CHO cells overexpressing mouse TIGIT (nM) |
|---|---|---|---|---|---|
| 55796-G1 | 0.96 | 1.32 | 2.96 | 0.24 | 1.50 |
| 55796-G1LALA | 0.84 | 1.34 | 3.36 | 0.37 | 1.56 |
| Tiragolumab | 1.35 | 4.18 | N.B. | 0.63 | NA |

| | | | | | |
|---|---|---|---|---|---|
| Note: "N.B.": no binding; and "NA": not available. | | | | | |

It could be seen from Table 8 and Figures 11 to 13 that, for the anti-TIGIT monoclonal antibody of the present invention, (1) its binding activity to the human TIGIT protein overexpressed on the surface of CHO cells was better than that of the control molecule Tiragolumab; (2) its binding activity to cynomolgus monkey TIGIT protein overexpressed on the surface of CHO cells was better than that of the control molecule Tiragolumab; and (3) it showed significant binding to the mouse TIGIT protein overexpressed on the surface of CHO cells.

It could be seen from Table 8 and Figures 14 to 15 that, for the anti-TIGIT monoclonal antibody of the present invention, (1) its ability to block the binding of human CD1 55 to the human TIGIT protein overexpressed on the surface of CHO cells was better than that of the control molecule Tiragolumab; (2) the anti-TIGIT antibody molecules bound to the mouse TIGIT protein overexpressed on the cell surface of CHO cells could significantly block the binding of mouse CD155 to the mouse TIGIT protein overexpressed on the surface of CHO cells.

### Test Example 3: Binding of anti-TIGIT monoclonal antibody to TIGIT on surface of primary T cells

The binding activity of the anti-TIGIT antibody of the present invention to TIGIT on the surface of activated T cells was detected based on the flow cytometry detection method.

Specifically, human PBMC were sorted according to the experimental protocol provided by STEMCELL (stemcell, Cat. No.: #17951C) to obtain human total T cells. The concentration of the T cells was adjusted to 1.0×10⁶ cells/mL using X-VIVO15 medium (purchased from lonza, Cat. No. 04-418Q), then 1µL of IL-2 stock solution (1 million IU) was added, while CD3/CD28 Dynabeads (purchased from gibco, No.: 11132D) was added at 1:1 (bead-to-cell), and cultured in a 5% CO₂ incubator at 37°C for 48 h. The activated T cells were adjusted to an appropriate cell density and added to a 96-well flow cytometry plate. After centrifugation, the gradiently diluted sample to be tested was added, and incubated at 4°C for 30 minutes. After washing twice with PBS, fluorescent secondary antibody correspondingly diluted to an appropriate concentration was added, incubated at 4°C for 30 minutes, and washed twice with PBS. The cells resuspended in PBS was added, and detected on a CytoFlex flow cytometer, and the corresponding MFI was calculated.

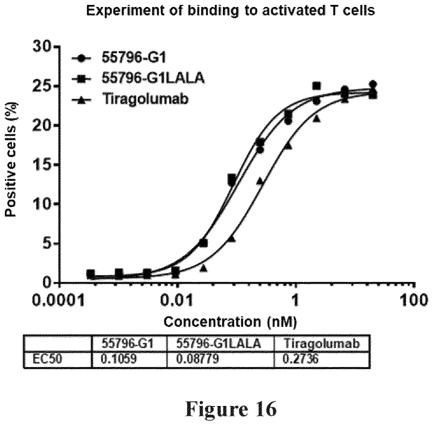

The results were shown in Figure 16. The results showed that the anti-TIGIT antibodies 55796-G1 and 55796-G1LALA of the present invention could bind to TIGIT molecules on the surface of T cells, and their binding activity was better than that of the control molecule Tiragolumab.

Although specific embodiments of the present invention have been described in detail, those skilled in the art will understand that according to all the teachings that have been disclosed, various modifications and substitutions can be made to these details, and these changes are within the protection scope of the present invention. The full scope of the present invention is given by the appended claims and any equivalents thereof.

## Claims

1. A bispecific antibody, which comprises:
a first protein functional region targeting TIGIT, and
a second protein functional region targeting a target (e.g., PD-L1) different from TIGIT;
wherein:
the first protein functional region is an anti-TIGIT immunoglobulin or an antigen-binding fragment thereof;
the heavy chain variable region of the anti-TIGIT immunoglobulin comprises HCDR1 with the amino acid sequence as set forth in SEQ ID NO: 10, HCDR2 with the amino acid sequence as set forth in SEQ ID NO: 11, and HCDR3 with the amino acid sequence as set forth in SEQ ID NO: 12; and
the light chain variable region of the anti-TIGIT immunoglobulin comprises LCDR1 with the amino acid sequence as set forth in SEQ ID NO: 13, LCDR2 with the amino acid sequence as set forth in SEQ ID NO: 14, and LCDR3 with the amino acid sequence as set forth in SEQ ID NO: 15.

2. The bispecific antibody according to claim 1, wherein the first protein functional region and the second protein functional region are directly linked or linked via a linker;
preferably, the linker is (GGGGS)ₘ, and m is a positive integer, such as 1, 2, 3, 4, 5 or 6;
preferably, the linker is (GGGGS)ₙG, and n is a positive integer, such as 1, 2, 3, 4, 5 or 6;
preferably, the amino acid sequence of the linker is set forth in SEQ ID NO: 2.

3. The bispecific antibody according to any one of claims 1 to 2, wherein the number of the first protein functional region and the second protein functional region are independently 1, 2, or more than 2.

4. The bispecific antibody according to any one of claims 1 to 3, wherein,
the first protein functional region is an anti-TIGIT immunoglobulin or an antigen-binding fragment thereof, and the second protein functional region is a single-domain antibody or a single-chain antibody targeting a target different from TIGIT;
preferably, the single-domain antibody is an anti-PD-L1 single-domain antibody;
preferably, the single-chain antibody is an anti-PD-L1 single-chain antibody.

5. The bispecific antibody according to any one of claims 1 to 4, wherein,
the first protein functional region is an anti-TIGIT single-chain antibody, and the second protein functional region is an immunoglobulin or an antigen-binding fragment thereof targeting a target different from TIGIT;
preferably, the immunoglobulin targeting a target different from TIGIT is an anti-PD-L1 immunoglobulin.

6. The bispecific antibody according to claim 5, wherein,
the anti-PD-L1 single-domain antibody comprises one heavy chain variable region, and the heavy chain variable region comprises HCDR1 with the amino acid sequence as set forth in SEQ ID NO: 16, HCDR2 with the amino acid sequence as set forth in SEQ ID NO: 17, and HCDR3 with the amino acid sequence as set forth in SEQ ID NO: 18;
preferably, the amino acid sequence of the anti-PD-L1 single-domain antibody is set forth in SEQ ID NO: 3.

7. The bispecific antibody according to any one of claims 1 to 6, wherein,
the heavy chain variable region of the anti-TIGIT immunoglobulin has an amino acid sequence set forth in SEQ ID NO: 19, and the light chain variable region of the anti-TIGIT immunoglobulin has an amino acid sequence set forth in SEQ ID NO: 20;
preferably,
the heavy chain of the anti-TIGIT immunoglobulin has an amino acid sequence set forth in SEQ ID NO: 1, and the light chain of the anti-TIGIT immunoglobulin has an amino acid sequence set forth in SEQ ID NO: 5.

8. The bispecific antibody according to any one of claims 1 to 7, wherein,
the constant region of the anti-TIGIT immunoglobulin or of the immunoglobulin targeting a target different from TIGIT is derived from a human antibody;
preferably, the constant region is selected from the group consisting of the constant regions of human IgGl, IgG2, IgG3 or IgG4.

9. The bispecific antibody according to any one of claims 1 to 8, wherein,
the heavy chain constant region of the anti-TIGIT immunoglobulin or of the immunoglobulin targeting a target different from TIGIT is human Ig gamma-1 chain C region (e.g., NCBI ACCESSION: P01857) or human Ig gamma-4 chain C region (e.g., NCBI ACCESSION: P01861.1), and the light chain constant region is human Ig kappa chain C region (e.g., NCBI ACCESSION: P01834);
preferably, the heavy chain constant region of the anti-TIGIT immunoglobulin further comprises a L234A mutation and a L235A mutation according to the EU numbering system.

10. The bispecific antibody according to any one of claims 4, and 6 to 9, wherein the single-domain antibody or single-chain antibody is linked to C-terminal or N-terminal of the anti-TIGIT immunoglobulin, for example, the number of the single-domain antibody or single-chain antibody is 2, and one end of each single-domain antibody or single-chain antibody is respectively linked to C-terminal or N-terminal of the two heavy chains of the anti-TIGIT immunoglobulin.

11. The bispecific antibody according to any one of claims 4, and 6 to 10, wherein the single-domain antibody is an anti-PD-L1 single-domain antibody, and the peptide chain obtained by linking the single-domain antibody to the anti-TIGIT immunoglobulin has an amino acid sequence as set forth in SEQ ID NO: 4.

12. The bispecific antibody according to any one of claims 4, and 6 to 11, which is a tetramer formed with two identical first peptide chains and two identical second peptide chains, wherein,
the amino acid sequence of the first peptide chain is set forth in SEQ ID NO: 4; and the amino acid sequence of the second peptide chain is set forth in SEQ ID NO: 5.

13. An isolated nucleic acid molecule, which encodes the bispecific antibody according to any one of claims 1 to 12.

14. A vector, which comprises the isolated nucleic acid molecule according to claim 13.

15. A host cell, which comprises the isolated nucleic acid molecule according to claim 13, or the vector according to claim 14.

16. A method of preparing the bispecific antibody according to any one of claims 1 to 12, comprising a step of culturing the host cell according to claim 15 under suitable conditions, and recovering the bispecific antibody from a cell culture.

17. A conjugate, which comprises a bispecific antibody and a coupling moiety, wherein the bispecific antibody is the bispecific antibody according to any one of claims 1 to 12, and the coupling moiety is a detectable label; preferably, the coupling moiety is a radioactive isotope, a fluorescent substance, a luminescent substance, a colored substance or an enzyme.

18. A kit, which comprises the bispecific antibody according to any one of claims 1 to 12, or the conjugate according to claim 17;
preferably, the kit further comprises a second antibody that is capable of specifically binding to the bispecific antibody; optionally, the second antibody further comprises a detectable label, such as a radioactive isotope, a fluorescent substance, a luminescent substance, a colored substance or an enzyme.

19. A pharmaceutical composition, which comprises the bispecific antibody according to any one of claims 1 to 12 or the conjugate according to claim 17; optionally, the pharmaceutical composition further comprises a pharmaceutically acceptable excipient.

20. Use of the bispecific antibody according to any one of claims 1 to 12 or the conjugate according to claim 17 in the manufacture of a medicament for the treatment or prevention of a malignant tumor; preferably, the malignant tumor is selected from the group consisting of melanoma, liver cancer, stomach cancer, renal cell cancer, ovarian cancer, colon cancer, breast cancer, esophageal cancer, and head and neck cancer.

21. The bispecific antibody according to any one of claims 1 to 12 or the conjugate according to claim 17 for use in treatment or prevention of a malignant tumor; preferably, the malignant tumor is selected from the group consisting of melanoma, liver cancer, gastric cancer, renal cell carcinoma, ovarian cancer, colon cancer, breast cancer, esophageal cancer, and head and neck cancer.

22. A method for treating or preventing a malignant tumor, comprising a step of administering to a subject in need thereof an effective amount of the bispecific antibody according to any one of claims 1 to 12 or the coupling agent of claim 17; preferably, the malignant tumor is selected from the group consisting of melanoma, liver cancer, gastric cancer, renal cell carcinoma, ovarian cancer, colon cancer, breast cancer, esophageal cancer, and head and neck cancer.
